(19) Europäisches Patentamt
European Patent Office
Office européen des brevets

(11) EP 2 588 585 B1

(12) # FASCICULE DE BREVET EUROPEEN

(45) Date de publication et mention de la délivrance du brevet:
**27.03.2019 Bulletin 2019/13**

(21) Numéro de dépôt: **11743276.5**

(22) Date de dépôt: **30.06.2011**

(51) Int Cl.:
***C11C 3/00*** *(2006.01)*

(86) Numéro de dépôt international:
**PCT/FR2011/051532**

(87) Numéro de publication internationale:
**WO 2012/001315 (05.01.2012 Gazette 2012/01)**

(54) **PROCÉDÉ DE FONCTIONNALISATION DE CORPS GRAS D'ORIGINE NATURELLE**

VERFAHREN ZUR FUNKTIONALISIERUNG NATÜRLICHER FETTSTOFFE

METHOD FOR FUNCTIONALIZING NATURAL FATTY SUBSTANCES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.06.2010 FR 1002738**

(43) Date de publication de la demande:
**08.05.2013 Bulletin 2013/19**

(73) Titulaires:
- **Centre National de la Recherche Scientifique (C.N.R.S.)**
  **75016 Paris (FR)**
- **Ecole Nationale Supérieure de Chimie de Montpellier**
  **34296 Montpellier Cedex 5 (FR)**
- **Université Montpellier 2 Sciences et Techniques**
  **34095 Montpellier Cedex 5 (FR)**

(72) Inventeurs:
- **CAILLOL, Sylvain**
  **F-34090 Montpellier (FR)**
- **BOUTEVIN, Bernard**
  **F-34090 Montpellier (FR)**
- **DESROCHES, Myriam**
  **F-34090 Montpellier (FR)**

(74) Mandataire: **Lavoix**
**62, rue de Bonnel**
**69448 Lyon Cedex 03 (FR)**

(56) Documents cités:
**WO-A2-02/100991 US-A- 4 566 878**

- **LLUCH CRISTINA ET AL: "Rapid Approach to Biobased Telechelics through Two One-Pot Thiol-Ene Click Reactions", BIOMACROMOLECULES, vol. 11, no. 6, 12 mai 2010 (2010-05-12), pages 1646-1653, XP002620574,**
- **BANTCHEV GRIGOR B ET AL: "Free Radical Addition of Butanethiol to Vegetable Oil Double Bonds", JOURNAL OF AGRICULTURAL AND FOOD CHEMISTRY, vol. 57, no. 4, février 2009 (2009-02), pages 1282-1290, XP002620575, ISSN: 0021-8561 cité dans la demande**
- **SAMUELSSON J ET AL: "Thiol-ene coupling reaction of fatty acid monomers", JOURNAL OF POLYMER SCIENCE, PART A: POLYMER CHEMISTRY 20041215 JOHN WILEY AND SONS INC. US, vol. 42, no. 24, 15 décembre 2004 (2004-12-15), pages 6346-6352, XP002621241, DOI: DOI:10.1002/POLA.20468**
- **LOWE, ANDREW B.: "Thiol-ene "click" reactions and recent applications in polymer and materials synthesis", POLYMER CHEMISTRY, vol. 1, no. 1, 25 novembre 2009 (2009-11-25), pages 17-36, XP002621242,**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 588 585 B1

## Description

La présente invention concerne un procédé de fonctionnalisation de corps gras d'origine naturelle et les corps gras fonctionnalisés ainsi obtenus. La présente invention concerne également l'utilisation des corps gras fonctionnalisés obtenus pour la préparation de polymères. Enfin l'invention concerne un procédé de préparation de polymères à partir d'au moins un corps gras fonctionnalisé obtenu par ledit procédé de fonctionnalisation.

Les huiles végétales et leurs dérivés peuvent faire l'objet de nombreuses modifications chimiques mettant en jeu les insaturations qu'ils présentent. Notamment, l'introduction de fonctions réactives permet d'utiliser ces huiles modifiées en tant que composés agro-sourcés dans la production de polymère, en remplacement de composés non naturels pouvant être toxiques.

Différentes techniques ont été décrites pour l'introduction de fonctions réactives sur les corps gras d'origine naturelle.

On peut notamment citer des réactions d'epoxidation suivies d'une hydrolyse permettant la préparation de corps gras fonctionnalisés par des alcools secondaires. Ce type de procédé a notamment été décrit dans la demande de brevet WO-2006/094227. Cette méthode reste la méthode la plus utilisée industriellement pour la fonctionnalisation des huiles. Cependant, les alcools secondaires présentent une réactivité limitée par rapport aux alcools primaires.

Il est donc important de fournir une méthode de fonctionnalisation de corps gras d'origine naturelle notamment par des fonctions alcool primaire.

L'hydroformylation des huiles, notamment décrite par A.Guo et al. (Journal of Polymers and The Environment, 2002, 10 (1/2), 49-52) donne accès à des fonctions aldéhyde qui après hydrogénation permet l'accès à des alcools primaires. Les réactions de Diels-Alder ont également été utilisées pour donner accès à des dérivés fonctionnels.

D'autres auteurs, notamment Sharma et al (Journal of Agricultural and Food Chemistry, 2006, 54 (26), 9866-9872 ; et US-2006/0009365) ont mis au point un procédé de production de corps gras fonctionnalisés comprenant une étape d'epoxidation suivie d'une étape d'addition de thiol, notamment butanethiol. Les composés obtenus sont décrits comme lubrifiant, notamment dans le domaine de l'automobile.

Tous ces procédés présentent l'inconvénient de nécessiter plusieurs étapes et parfois des réactifs ou catalyseurs coûteux et souvent toxiques.

Des études ont été menées sur la modification des doubles liaisons des corps gras par la réaction thiol-ène. On peut notamment citer la publication de Samuelsson et al (Journal of Polymer Science, Part A : Polymer Chemistry, 2004, 42, 6346-6352) qui décrit la réaction de couplage thiol-ène sur des monomères acides gras. Bantchev et al (Journal of Agricultural and Food Chemistry, 2009, 57(4), 1282-1290) divulguent également la réaction thiol-ène sur des huiles végétales et l'utilisation du produit obtenu comme lubrifiant.

Cependant, ces deux procédés ne mettent pas en oeuvre des thiols fonctionnalisés et ne permettent donc pas l'introduction de fonctions réactives sur les corps gras d'origine naturelle. Les corps gras obtenus par ces procédés ne peuvent pas être utilisés dans la production de polymères, notamment par polycondensation.

Par ailleurs, Lluch et al. (Biomacromolecules, vol. 11, n°6, 12 mai 2010) décrit l'application de la réaction thiol-ène à la préparation de polymères téléchéliques qui sont des polymères capables de subir une polymérisation ultérieure du fait de la présence de groupements réactions ; et Lowe et al. (polymer chemistry, vol. 1, n°1, 25 novembre 2009) décrit l'application de la réaction thiol-ène, et en particulier la réaction entre une huile végétale et le butanethiol.

WO 02/100991 décrit la réaction d'une trioléine, d'une huile de riz ou de bourrache avec du 2-mercapoéthanol et de l'AIBN comme amorceur thermique pour préparer un corps fonctionnalisé. Enfin, Bantchev et al. (Journal of Adricultural and Food Chemistry, vol.57, n°4, 2009) décrit la préparation d'un corps gras fonctionnalisé à partir d'un acide gras insaturé avec du butanethiol.

Il est nécessaire de fournir un procédé permettant, en une seule étape, d'introduire des fonctions réactives sur les corps gras d'origine naturelle.

Un des objectifs de l'invention est de fournir un procédé, simple et peu coûteux, de fonctionnalisation d'un corps gras d'origine naturelle en une seule étape. L'invention permet ainsi la préparation de dérivés polyfonctionnels en une seule étape, en évitant les inconvénients des méthodes antérieures.

Un autre objectif de l'invention est de produire des produits issus de réactifs agro-sourcés et porteurs de fonctions réactives, notamment alcool, acide, etc.

Un autre objet encore de l'invention est de fournir des synthons de type corps gras fonctionnalisés d'origine naturelle pour la synthèse de polymère.

Un autre objectif de l'invention est de fournir un nouveau procédé de synthèse de polymère partiellement ou totalement issus de réactifs biosourcés.

L'invention concerne un procédé de préparation, en une seule étape, d'un corps gras fonctionnalisé par des fonctions réactives. Le procédé de l'invention met en oeuvre une réaction de type thiol-ène.

L'invention concerne l'application de la réaction thiol-ène pour l'introduction de fonctions réactives au sein de corps gras d'origine naturelle.

La présente invention concerne un procédé de préparation d'un corps gras fonctionnalisé comprenant la réaction d'un corps gras d'origine naturelle choisi parmi

- les huiles végétales comprenant au moins deux insaturations et leurs dérivés,
- les acides gras comprenant au moins une insaturation et leurs dérivés,
- leurs mélanges
  avec un dérivé thiol de formule (I)

$$G_n\text{-}L^1\text{-}L^2\text{-}SH \qquad (I)$$

dans laquelle

- G, identique ou différent, représente $-NR^1H$ ; -C(O)OH ;
- n représente 1 ou 2 ;
- $R^1$ représente un atome d'hydrogène ; un radical alkyle, linéaire ou ramifié, en $C_1$-$C_{10}$, de préférence en $C_1$-$C_6$, de préférence méthyle, éthyle, non substitué ou substitué par au moins un groupe choisi parmi -OH, -C(O)OH, $-NH_2$, -C(O)H,

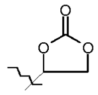
;

- $L^1$ représente un groupe $-CH_2$ ou une liaison directe ;
- $L^2$ représente

  - un alkyle, linéaire ou ramifiée, en $C_1$-$C_{20}$, de préférence $C_1$-$C_{12}$, de préférence méthyle, éthyle, propyle, butyle, pentyle, hexyle, heptyle, octyle, nonyle, décyle, undécyle, dodécyle, comprenant éventuellement un ou plusieurs hétéroatomes choisis parmi un atome d'oxygène, un atome d'azote, un atome de soufre, non-substitué ou substitué par au moins un groupe choisi parmi -OH, -C(O)OH, -C(O)H,

  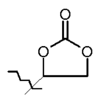
  ,

  $-NHR^2$ avec $R^2$ représente un atome d'hydrogène ; un radical alkyle, linéaire ou ramifié, en $C_1$-$C_{10}$, de préférence en $C_1$-$C_6$, de préférence méthyle, éthyle, non substitué ou substitué par au moins un groupe choisi parmi -OH, -C(O)OH, $-NH_2$, -C(O)H,

  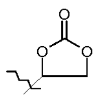
  ;

  - un carbo ou hétérocycle en $C_3$ à $C_8$, en particulier un groupe cycloaliphatique, un groupe aryle, de préférence phényle, un groupe hétéroaryle, de préférence pyridine ;
  - sous action d'un rayonnement UV, ou
  - sous action d'un rayonnement UV et en présence d'un photoamorceur.

Selon l'invention, l'huile végétale et ses dérivés comprennent entre 2 et 20 insaturations. L'huile végétale peut être choisie parmi les huiles végétales naturelles brutes ou purifiées et les huiles végétales issues de plantes ou de cultures génétiquement modifiées.
Selon l'invention l'huile végétale est choisie parmi l'huile de canola, l'huile de carthame, l'huile de colza, l'huile de coton, l'huile de lin, l'huile de maïs, l'huile de noisette, l'huile de noix de coco, l'huile d'olive, l'huile de palme, l'huile de pépins de raisins, l'huile de ricin, l'huile de sésame, l'huile de soja, l'huile de tournesol, seules ou en mélange.
Les dérivés d'huiles végétales peuvent être choisis parmi les esters gras obtenus par estérification ou transestérification d'huile végétale, notamment les huiles végétales selon l'invention ; des amides grasses obtenues par amidification ou

transamidification d'huile végétale, notamment les huiles végétales selon l'invention et des huiles végétales, notamment les huiles végétales selon l'invention, partiellement époxydées.

On entend par huiles végétales partiellement époxydées des huiles végétales pour lesquelles une partie des insaturations a été époxydée et dont au moins deux insaturations n'ont pas réagi, de préférence entre 20 et 80%, de préférence entre 30 et 50% des insaturations sont époxydées.

De manière générale, on entend par (trans)estérification et (trans)amidification des réactions qui ont lieu sur la fonction terminale des triglycérides constitutifs de l'huile végétale et on entend par epoxidation des réactions qui ont lieu sur les insaturations de l'huile végétale.

Selon l'invention, les acides gras et leurs dérivés comprennent entre 1 et 6 insaturations. Selon l'invention, les acides gras sont choisis parmi l'acide arachidonique, l'acide docosahexaénoique, l'acide éicosapentaénoique, l'acide érucique, l'acide linoléique, l'acide linolénique, l'acide nervonique, l'acide oléique, l'acide palmitoléique, l'acide ricinoléique, l'acide vernolique. Les acides gras peuvent également être obtenus à partir des huiles végétales selon l'invention.

Les dérivés d'acides gras sont notamment choisis parmi les esters d'acides gras, par exemple oléate de méthyle, les amides grasses obtenues par amidification des acides gras, et les thioesters gras issus de la thioestérification des acides gras, notamment des acides gras selon l'invention.

De manière préférée, l'invention concerne un procédé pour lequel G, identique ou différent, représente $-NR^1H$ ; -C(O)OH ; $R^1$ étant tel que défini ci-dessus.

De manière également préférée, l'invention concerne un procédé pour lequel G identique ou différent, représente -C(O)OH, $-NH_2$.

De manière préférée n représente 1.

De manière préférée, l'invention concerne un procédé pour lequel $L^2$ représente un phényl ou une chaine alkyle linéaire ou ramifiée, en $C_1$-$C_{20}$, de préférence en $C_1$-$C_{12}$, de préférence méthyle, éthyle, propyle, butyle, pentyle, hexyle, heptyle, octyle, nonyle, décyle, undécyle, dodécyle.

Selon l'invention, l'invention concerne un procédé pour lequel le dérivé thiol de formule (I) est choisi parmi la cystéamine (2-aminoethanethiol), l'acide thioglycolique (acide mercaptoacétique), l'acide mercaptosuccinique (acide thiomalic), l'acide 3-mercaptopropionique,la cystéine, 4-aminothiophenol, l'acide 6-mercaptohexanoique, l'acide 3-mercaptobenzoique, l'acide thiosalicylique, l'acide 4-mercaptophenylacetique, l'acide 8-mercaptooctanoique, l'acide 12-mercaptododecanoique,.

| | |
|---|---|
| cystéamine | 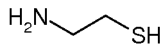 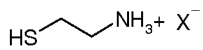 |
| acide thioglycolique | 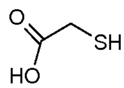 |
| β-mercaptoethanol | 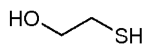 |
| acide mercaptosuccinique | 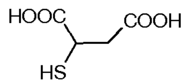 |
| acide 3-mercaptopropionique | 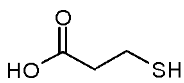 |
| 1-thiolglycérol | 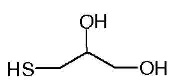 |
| cystéine | 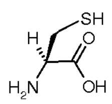 |
| alcool 2-mercaptobenzylique | 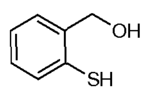 |

(suite)

| | |
|---|---|
| acide 4-mercaptophenylacetique | 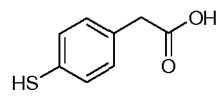 |
| 3-mercapto-1-propanol | 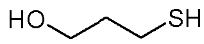 |
| 4-mercapto-1-butanol | 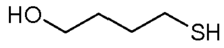 |
| 4-mercaptophénol | 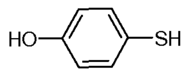 |
| 4-aminothiophénol | 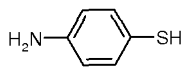 |
| acide 6-mercaptohexanoïque | 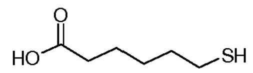 |
| acide 3-mercaptobenzoïque | 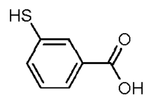 |
| acide thiosalycylique | 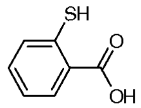 |
| acide 8-mercaptooctanoïque | 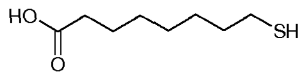 |
| 9-mercapto-1-nonanol | 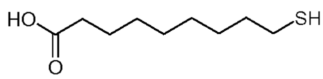 |
| acide 12-mercaptododécanoïque | 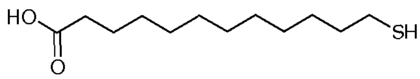 |
| 3-mercaptobutanal | 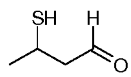 |
| 3-mercaptohexanal | 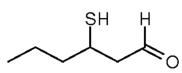 |

Selon l'invention, le procédé peut être mené à une température comprise entre 0°C et la température de dégradation totale du corps gras d'origine naturelle et en présence d'un composé amorceur thermique ou d'un amorceur redox.

On entend par température de dégradation totale du corps gras d'origine naturelle, la température au-delà de laquelle toutes les insaturations dudit corps gras ont été hydrogénées. Une telle température dépend du corps gras, elle est connue ou peut être déterminée.

De manière préférée, la température est comprise entre la température ambiante et la température de dégradation totale du corps gras d'origine naturelle, de préférence elle est comprise entre la température ambiante et 250°C, plus préférentiellement entre 40°C et 150°C.

On entend par amorceur thermique tout type d'amorceur générant des radicaux par décomposition thermique. On peut notamment citer azobisisobutyronitrile (AIBN), peroxyde de benzoyle, tert-amyl peroxypivalate, boranes, bis(4-tert-butylcyclohexyl)peroxydicarbonate.

On entend par amorceur redox tout type d'amorceur pour lequel la production de radicaux résulte d'une réaction d'oxydo-réduction. On peut notamment citer le système tert-butylperbenzoate/acide erythorbique, peroxyde de benzoyle/amine,

$H_2O_2/Fe^{2+}$ (réactif de Femton).
Le choix de l'amorceur dépend de la température à laquelle le procédé est mis en oeuvre et pourra être déterminé suivant les conditions réactionnelles.

On peut notamment citer, sans y être limiter, l'utilisation de l'AIBN à une température de 85°C, ou encore l'utilisation de tert-amyl peroxypivalate à une température de 50°C.

Selon l'invention le procédé peut être mené sous rayonnement UV et éventuellement en présence d'un amorceur photochimique (photoamorceur).
On entend par amorceur photochimique ou photoamorceur tout type d'amorceur générant des radicaux sous l'action des rayons UV. On peut citer à titre d'exemple le benzyle.
Le choix de l'amorceur dépend de la longueur d'onde du rayonnement UV utilisé et pourra être déterminé suivant les conditions réactionnelles.

De manière générale, la longueur d'onde du rayonnement UV est comprise entre 200 et 800 nm, de préférence entre 250 et 500 nm.

De préférence, le procédé de l'invention est mis en oeuvre sous action d'un rayonnement UV ou sous action d'un rayonnement UV et en présence d'un photoamorceur.
De manière avantageuse, le procédé de l'invention est mis en oeuvre sous action d'un rayonnement UV.
Le procédé de l'invention peut être réalisé en masse ou en solvant. Le solvant est utilisé dans les systèmes pouvant présentés des problèmes de solubilisation, notamment solubilisation de l'amorceur.
Le procédé selon l'invention peut être réalisé en présence d'un solvant qui, dans le cas où le procédé est effectué sous rayonnement UV, doit être transparent aux UV.
Les solvants pouvant être utilisés dans le procédé à température et en présence d'un amorceur thermique sont notamment choisis parmi tétrahydrofuranne, toluène, pentane, hexane, heptane, cyclohexane, méthanol, éthanol, acétate d'éthyle, diéthyl éther, dioxane, chloroforme, acétonitrile, dichlorométhane, dichloroéthane, eau, solvants verts de type succinates, seuls ou en mélange.
Les solvants pouvant être utilisés dans le procédé sous rayonnement UV sont notamment choisis parmi méthanol, éthanol.

Pour le procédé de l'invention les caractéristiques suivantes peuvent être combinées de manière totale ou partielle:

- G représente $-NR^1H$; -C(O)OH ;
- $L^1$ représente un groupe $-CH_2$ ; une liaison directe ;
- $L^2$ représente :

  - un alkyle, linéaire ou ramifiée, en $C_1$-$C_{20}$, de préférence $C_1$-$C_{12}$, de préférence méthyle, éthyle, propyle, butyle, pentyle, hexyle, heptyle, octyle, nonyle, décyle, undécyle, dodécyle, comprenant éventuellement un ou plusieurs hétéroatomes choisis parmi un atome d'oxygène, un atome d'azote, un atome de soufre, non-substitué ou substitué par au moins un groupe choisi parmi -OH, -C(O)OH, -C(O)H,

    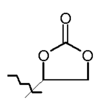 , -

    $NHR^2$ avec $R^2$ représente un atome d'hydrogène ; un radical alkyle, linéaire ou ramifié, en $C_1$-$C_{10}$, de préférence en $C_1$-$C_6$, de préférence méthyle, éthyle, non substitué ou substitué par au moins un groupe choisi parmi -OH, -C(O)OH, $-NH_2$, -C(O)H,

    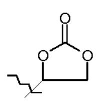 ;

  - un carbo ou hétérocycle en $C_3$ à $C_8$, en particulier un groupe cycloaliphatique, un groupe aryle, de préférence phényle, un groupe hétéroaryle, de préférence pyridine ;
  - température comprise entre 0°C et la température de dégradation totale du corps gras d'origine naturelle, de préférence de préférence entre la température ambiante et la température de dégradation totale du corps gras d'origine naturelle, de préférence entre la température ambiante et 250°C, de préférence entre 40°C et 150°C et un amorceur thermique ou rédox ;

- mise en oeuvre d'un rayonnement UV ;
- mise en oeuvre d'un rayonnement UV et d'un amorceur photochimique.

De manière avantageuse, l'invention concerne un procédé pour lequel G est $NHR^1$, avec $R^1$ selon l'invention, de préférence $R^1$ représente H, et le procédé est conduit sous rayonnement UV.

De manière également avantageuse, l'invention concerne un procédé pour lequel G est - C(O)OH et le procédé est conduit à une température comprise entre 0°C et la température de dégradation totale du corps gras d'origine naturelle, de préférence entre la température ambiante et la température de dégradation totale du corps gras d'origine naturelle, de préférence entre la température ambiante et 250°C, de préférence entre 40°C et 150°C.

Le procédé de l'invention présente l'avantage de ne pas nécessiter d'étapes de purification multiples et poussées. A titre d'exemple, une simple purification par extraction liquide/liquide ou une distillation à pression réduite et faible température suffit à obtenir le corps gras fonctionnalisé à un degré de purification élevé.

Le procédé de la présente invention permet avantageusement un taux de conversion et de fonctionnalisation des corps gras d'origine naturelle élevé, notamment compris entre 25 et 100%, de préférence compris entre 50 et 100%.

Un autre avantage du procédé selon l'invention est qu'il permet de contrôler le taux de fonctionnalisation moyen du corps gras d'origine naturelle en agissant par exemple sur la nature de l'huile, et donc le taux d'insaturations, ou sur la quantité de thiol fonctionnel mis en jeu. Il est ainsi possible d'adapter le procédé à l'utilisation qui va être faite des produits obtenus. Notamment, la diversité des huiles d'origine végétales permet de choisir l'huile utilisée en fonction de la fonctionnalisation recherchée. En effet, pour chaque type d'huile le nombre moyen d'insaturation est connu. Le tableau 1 donne un exemple des huiles pouvant être utilisées dans le procédé de l'invention et de leur nombre d'insaturations.

| Huiles végétales | Acide gras (%) | | | | | | | | | | |
|---|---|---|---|---|---|---|---|---|---|---|---|
| | 16:0 | 18:0 | 20:0 | 22:0 | 18:1 | 18:2 | 18:3 | 18:1 (12OH) | 22:1 | Saturés | Insaturés |
| Mais | 12 | 2 | | | 25 | 53 | | | | 21 | 79 |
| Cotton | 28 | 1 | | | 15 | 56 | | | | 29 | 71 |
| Lin | 5 | 2 | | | 20 | 17 | 56 | | | 7 | 93 |
| Lin (mutagénèse) | 6 | 3 | | | 15 | 73 | 3 | | | 9 | 91 |
| Noisette | 12 | 2 | 1 | 2 | 49 | 33 | 1 | | | 17 | 83 |
| Colza | 3 | 1 | | | 64 | 23 | 8 | | 1 | 4 | 96 |
| Canola | 4 | 2 | | | 66 | 19 | 9 | | | 6 | 94 |
| Carthame | 9 | 1 | | | 12 | 78 | | | | 10 | 90 |
| Sésame | 13 | 4 | | | 53 | 30 | | | | 17 | 83 |
| Soja | 13 | 3 | | | 23 | 55 | 6 | | | 16 | 84 |
| Soja (génétique) | 7 | 4 | | | 85 | 1 | 2 | | | 12 | 88 |
| Tournesol | 6 | 3 | | | 17 | 74 | | | | 9 | 91 |
| Tournesol (mutagénèse) | 3 | 2 | | | 92 | 2 | | | | 6 | 94 |
| Ricin | | | | | 2 | 6 | 1 | 89 | | 2 | 98 |
| Palme | 40 | 5 | | | 46 | 9 | | | | 45 | 55 |
| Olive | 11 | 2 | | | 78 | 9 | | | | 13 | 87 |
| Pépin raisins | 8 | 4 | | | 17 | 76 | | | | 12 | 88 |
| Noix coco | 8 | 3 | | | 7 | 1 | | | | 92 | 8 |

Tableau 1

Les acides gras sont définis par deux valeurs, la première représentant le nombre total de carbones constituant la chaîne, la deuxième étant le nombre de doubles liaisons présentes sur cette chaîne.

16:0=acide palmitique, 18:0=acide stéarique, 20:0=acide arachidique, 22:0=acide behenique, 18:1=acide oléique, 18:2=acide linoléique, 18:3=acide linolénique, 18:1(12OH)=acide ricinoléique, 22:1=acide érucique.

L'invention concerne également un corps gras fonctionnalisé susceptible d'être obtenu par le procédé selon l'invention.

L'invention concerne donc également un corps gras fonctionnalisé de formule (IIa), (IIb) ou (IIc) :

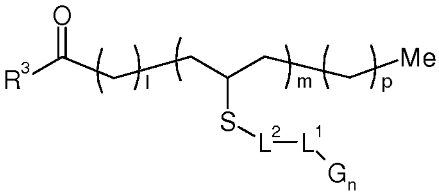

(IIa)

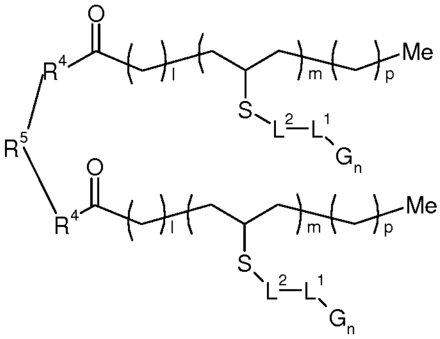

(IIb)

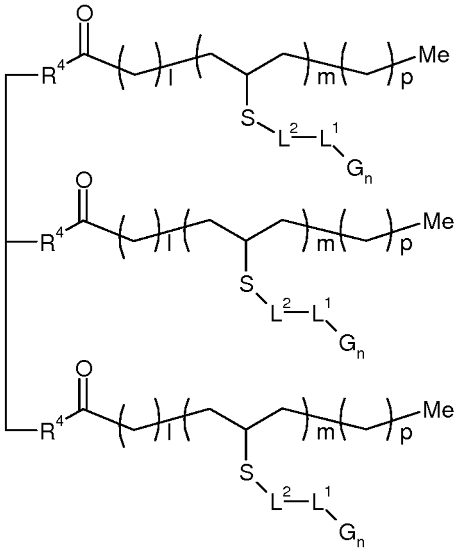

(IIc)

dans lesquelles :

- G, identique ou différent, représente $-NR^1H$ ; -C(O)OH ;
- n représente 1 ou 2 ;
- $R^1$ représente un atome d'hydrogène ; un radical alkyl, linéaire ou ramifié, en $C_1$-$C_{10}$, non substitué ou substitué par au moins un groupe choisi parmi -OH, -C(O)OH, $-NH_2$, - C(O)H,

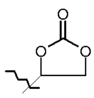 ;

de préférence $R^1$ représente un atome d'hydrogène ;
- $L^1$ représente un groupe $-CH_2$ ; une liaison directe ;
- $L^2$ représente
  - un alkyle, linéaire ou ramifiée, en $C_1$-$C_{20}$, de préférence $C_1$-$C_{12}$, de préférence méthyle, éthyle, propyle, butyle, pentyle, hexyle, heptyle, octyle, nonyle, décyle, undécyle, dodécyle, comprenant éventuellement un ou plusieurs hétéroatomes choisis parmi un atome d'oxygène, un atome d'azote, un atome de soufre, non-substitué ou substitué par au moins un groupe choisi parmi -OH, -C(O)OH, -C(O)H,

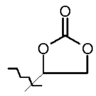
,

-$NHR^2$ avec $R^2$ représente un atome d'hydrogène ; un radical alkyle, linéaire ou ramifié, en $C_1$-$C_{10}$, de préférence en $C_1$-$C_5$, de préférence méthyle, éthyle ..., non substitué ou substitué par au moins un groupe choisi parmi -OH, -C(O)OH, -$NH_2$, -C(O)H,

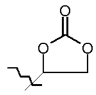
;

- un carbo ou hétérocycle en $C_3$ à $C_8$, en particulier un groupe cycloaliphatique, un groupe aryle, de préférence phényle, un groupe hétéroaryle, de préférence pyridine ;

• $R^3$ représente :

- un groupe hydroxyle (OH),
- un groupe alkoxy de type -OX, avec X choisi parmi alkyl, linéaire ou ramifié, en $C_1$-$C_{20}$, de préférence en $C_1$-$C_{12}$, éventuellement substitué par au moins un groupe choisi parmi -OH, -C(O)OH, -$NH_2$, -C(O)H,

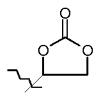
,

de préférence méthyle, éthyle, éthylène glycol, glycérol.
- un groupe amine -NY'Y", avec Y' et Y", identiques ou différents représentent un atome d'hydrogène, un alkyl linéaire ou ramifié, en $C_1$-$C_{20}$, de préférence $C_1$-$C_{12}$, éventuellement substitué par au moins un groupe choisi parmi -OH, -C(O)OH, -$NH_2$,-C(O)H,

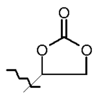
,

de préférence méthyle, éthyle, éthanolamine, diéthanolamine ; ou éthylènediamine, 1,3-diaminopropane, hexaméthylènediamine.
- un groupe thio de type SZ, avec Z choisi parmi un alkyl linéaire ou ramifié, en $C_1$-$C_{20}$, de préférence en $C_1$-$C_{12}$, éventuellement substitué par au moins un groupe choisi parmi -OH, -C(O)OH, -$NH_2$, -C(O)H,

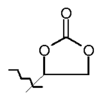
,

de préférence cystéamine (2-aminoethanethiol), acide thioglycolique (acide mercaptoacétique), β-mercaptoéthanol, acide mercaptosuccinique (acide thiomalic), acide 3-mercaptopropionique, 1-thiolglycérol (3-mercapto-1,2-propanediol), cystéine, 4-aminothiophenol, acide 6-mercaptohexanoique, l'acide 3-mercaptobenzoique, l'acide thiosalicylique, acide 4-mercaptophenylacetique, acide 8-mercaptooctanoique, 9-mercapto-1-nonanol, acide 12-mercaptododecanoique, 3-mercaptobutanal, 3-mercaptohexanal ;

• $R^4$ représente :

- un atome d'oxygène ;
- un atome d'azote ;

- $R^5$ représente :

  - un groupe alkyl linéaire ou ramifié, en $C_1$-$C_{20}$, de préférence en $C_1$-$C_{12}$, éventuellement substitué par au moins un groupe choisi parmi -OH, -C(O)OH, -$NH_2$,-C(O)H,

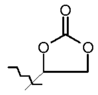

;

de préférence méthyle, éthyle, propyle, propanol ;

- l représente un nombre entier compris entre 1 et 10 ;
- m représente un nombre entier compris entre 1 et 3 ;
- p représente un nombre entier compris entre 0 et 10,

De manière préférée, l'invention concerne un corps gras fonctionnalisé de formule (IIa), (IIb) ou (IIc), dans lequel G, identique ou différent, représente -$NR^1H$ ; -C(O)OH ; $R^1$ étant tel que défini ci-dessus.
De manière préférée, l'invention concerne un corps gras fonctionnalisé de formule (IIa), (IIb) ou (IIc), dans lequel G identique ou différent représente -C(O)OH, -$NH_2$, -.
De manière préférée, l'invention concerne un corps gras fonctionnalisé de formule (IIa), (IIb) ou (IIc), dans lequel n représente 1.
De manière préférée, l'invention concerne un corps gras fonctionnalisé de formule (IIa), (IIb) ou (IIc), dans lequel $L^2$ représente un phényl ou une chaine alkyle linéaire ou ramifiée, en $C_1$-$C_{20}$, de préférence en $C_1$-$C_{12}$, de préférence méthyle, éthyle, propyle, butyle, pentyle, hexyle, heptyle, octyle, nonyle, décyle, undécyle, dodécyle.
Est également décrit un composé de formule (IIa) selon l'invention à l'exception des composés de formules (A) et/ou (B)

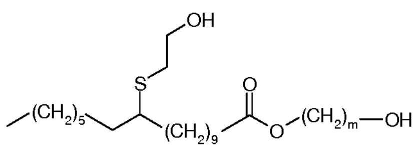

(A)

dans laquelle m représente un entier allant de 1 à 18, notamment de 1 à 12 ;

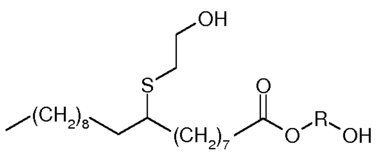

(B)

dans laquelle R représente $C_3H_6$, $C_4H_8$, $C_5H_{10}$ ou $C_6H_{12}$.
Est également décrit un composé de formule (IIa) selon l'invention à l'exception du composé de formule (C) :

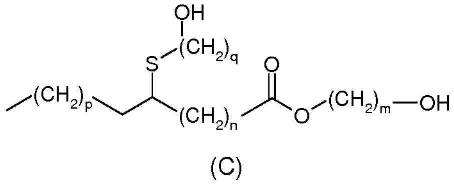

(C)

dans laquelle :

m représente un entier allant de 1 à 18, notamment de 1 à 12 ;
n représente un entier allant de 2 à 11 ;
p représente un entier allant de 1 à 10 ; et
q représente un entier allant de 1 à 18, notamment de 1 à 13.

De manière préférée, l'invention concerne un composé de formule (IIa) selon l'invention à l'exception de ceux pour lesquels n représente 1, G représente -OH, $L^1$ représente une liaison directe ou un groupe $-CH_2-$, $L^2$ représente un alkyle en $C_1$ à $C_{18}$, en particulier en $C_1$ à $C_{12}$, $R^3$ représente un groupe alkoxy de formule -OX dans laquelle X représente un radical alkyl en $C_1$ à $C_{18}$, en particulier en $C_1$ à $C_{12}$ substitué par un groupe -OH en position terminale, I représente un entier allant de 1 à 10 ; p représente un entier allant de 1 à 10 , m représente 1.

De manière préférée, l'invention concerne un composé de formule (IIa) selon l'invention à l'exception de ceux pour lesquels $R^3$ représente un groupe alkoxy de formule -OX dans laquelle X représente un radical alkyl en $C_1$ à $C_{18}$, en particulier en $C_1$ à $C_{12}$ substitué par un groupe -OH en position terminale substitué par un groupe -OH en position terminale.

Est également décrit concerne un composé de formule (IIb) selon l'invention à l'exception des composés de formules (D), (E), (F) et/ou (G)

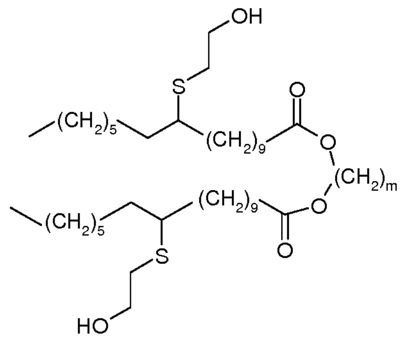

(D)

pour laquelle m représente un entier allant de 1 à 18, notamment de 1 à 12 ;

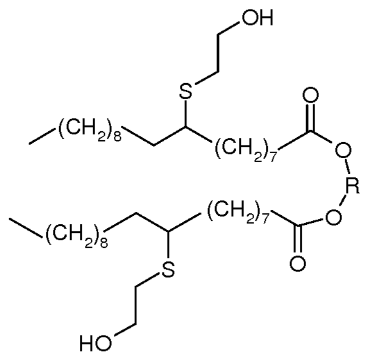

(E)

pour laquelle R représente $C_3H_6$, $C_4H_8$, $C_5H_{10}$, $C_6H_{12}$ ;

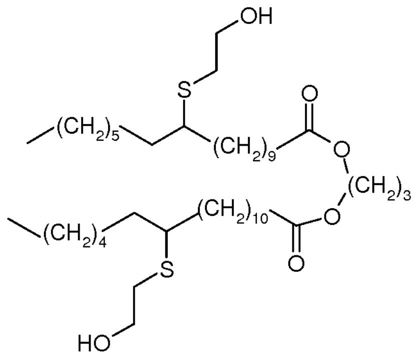
;
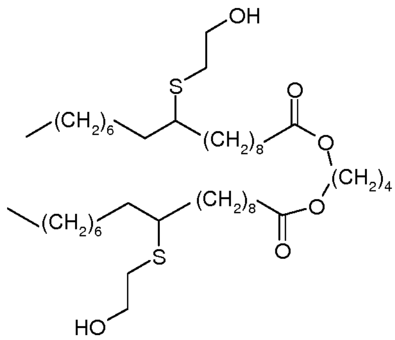
.

(F) (G)

De manière préférée, l'invention concerne un composé de formule (IIb) selon l'invention à l'exception du composé de formule (H)

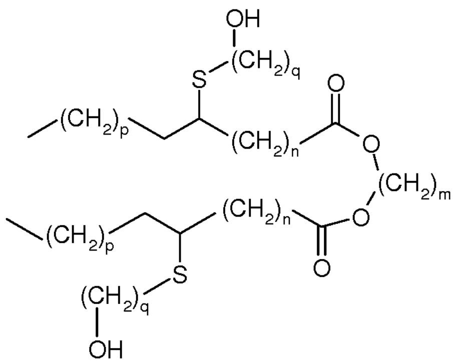

(H)

pour laquelle m représente un entier allant de 1 à 18, notamment de 1 à 12 ;

n représente un entier allant de 2 à 11 ;
p représente un entier allant de 1 à 10 ; et
q représente un entier allant de 1 à 18, notamment de 1 à 13.

De manière préférée, l'invention concerne un composé de formule (IIb) selon l'invention à l'exception des composés pour lesquels n représente 1, m représente 1, G représente - OH, $L^1$ représente une liaison directe ou un groupe $-CH_2-$, $L^2$ représente un alkyle en $C_1$ à $C_{18}$, en particulier en $C_1$ à $C_{12}$, I représente un entier allant de 1 à 10, p représente un entier allant de 1 à 10, $R^5$ représente un alkyl et $R^4$ un oxygène.

Le corps gras fonctionnalisé, soit susceptible d'être obtenu par le procédé de l'invention soit le corps gras fonctionnalisé de l'invention, comprend des groupements G réactifs. Il peut ainsi être avantageusement utilisé pour la préparation de polymères par polycondensation.

Un autre objet de l'invention concerne un procédé (a) de préparation d'un polymère comprenant:

(a1) la préparation d'un corps gras fonctionnalisé selon le procédé de l'invention,
(a2) la polycondensation entre le corps gras fonctionnalisé obtenu à l'étape (a1) et au moins une molécule au moins bifonctionnelle choisie parmi les isocyanates, alcools, acides carboxyliques, aminés, carbamates, aldéhydes, époxy.

A titre d'exemple, on peut citer les combinaisons suivantes :

| Corps gras fonctionnalisés (a1) | Molécule au moins difonctionnelle | Polymère obtenu |
|---|---|---|
| G=OH | C(O)OH | polyester |
| G=$NH_2$ | C(O)OH | polyamide |

(suite)

| Corps gras fonctionnalisés (a1) | Molécule au moins difonctionnelle | Polymère obtenu |
|---|---|---|
| G=$NH_2$ | 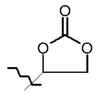 | polyuréthane |
| G=$NH_2$ | 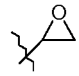 | résine époxy |
| G=OH | NC(O) | polyuréthane |

Un autre objet de l'invention concerne un procédé (b) de préparation d'un polymère comprenant :

(b1) la préparation d'un corps gras fonctionnalisé selon le procédé de l'invention,
(b2) la préparation d'un second corps gras fonctionnalisé, selon le procédé de l'invention, différent et au sein duquel la fonction G est différente de celle du corps gras préparé à l'étape (b1), puis
(b3) la polycondensation entre les deux corps gras fonctionnalisés.

A titre d'exemple, on peut citer les combinaisons suivantes :

| Corps gras fonctionnalisés (b1) | Corps gras fonctionnalisés (b2) | Polymère obtenu |
|---|---|---|
| G=OH | G=C(O)OH | polyester |
| G=$NH_2$ | G= C(O)OH | polyamide |
| G=$NH_2$ | G= 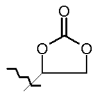 | polyuréthane |

L'invention concerne également l'utilisation, soit d'un corps gras fonctionnalisé susceptible d'être obtenu par le procédé de l'invention soit d'un corps gras fonctionnalisé de l'invention, pour la préparation d'un polymère.
Selon l'invention il est possible de préparer un polymère choisi parmi les polyesters ; polyamides ; polycarbamates ou polyisocyanates, notamment polyuréthanes ; les résines époxy ; les polycarbonates.
De manière avantageuse, l'utilisation soit de corps gras fonctionnalisés susceptibles d'être obtenus selon l'invention soit de corps gras fonctionnalisés de l'invention, peut permettre de conférer de la souplesse au polymère obtenu par polycondensation.
De manière avantageuse, l'utilisation, soit de corps gras fonctionnalisés susceptibles d'être obtenus selon l'invention soit de corps gras fonctionnalisés de l'invention, peut permettre une diminution voire une élimination totale des COV (Composés Organiques Volatils) lors de la préparation de polymère en comparaison avec les composés habituellement utilisés pour la production de tels polymères.

La figure 1 représente la courbe ATG (5°C/min, 10°C/min et 20°C/min) d'un polyol commercial (Desmophen ® 1150).
La figure 2 représente la courbe ATG (5°C/min, 10°C/min et 20°C/min) d'un polyol obtenu par le procédé de l'invention.

L'invention va maintenant être décrite à l'aide d'exemples non limitatifs.

Exemple 1 : Synthèse d'un corps gras fonctionnalisé de type polyols par voie photochimique à partir d'huile de colza (ne faisant pas partie de l'invention)

Dans un tube en quartz de 10 mL sont introduits l'huile de colza (2g, $2,3.10^{-3}$mol, soit environ $8,6.10^{-3}$mol d'insaturations) et le β-mercaptoéthanol (2,2g, $2,8.10^{-2}$mol). Le milieu réactionnel est placé sous irradiation UV d'une lampe mercure équipée d'un filtre 250-450 nm, délivrant une intensité de 15000mW/$cm^2$. Une forte agitation est maintenue pendant toute la durée de la réaction. Après 5 heures d'exposition, le milieu réactionnel est dilué dans l'acétate d'éthyle et l'excès de β-mercaptoéthanol est lavé trois fois avec une solution aqueuse de chlorure de sodium. La phase

organique est alors séchée par un agent desséchant de type sulfate de magnésium, puis concentrée à l'évaporateur rotatif sous vide à 40°C. Le polyol est obtenu sous forme d'un liquide visqueux orangé.

L'absence de signal à 5,40ppm ($C\underline{H}=C\underline{H}$) et à 2,00ppm ($C\underline{H}_2-CH=$) sur le spectre RMN $^1H$ du polyol indique que les doubles liaisons de l'huile de colza ont réagi. Ces disparitions s'accompagnent de l'apparition des signaux caractéristiques de l'addition du mercaptoéthanol, avec notamment l'apparition du multiplet à 2,5ppm caractéristique du proton appartenant au carbone asymétrique en alpha du soufre ($C\underline{H}-S$) d'un multiplet à 1,53ppm ($C\underline{H}_2-CH-S$) et à 1,40ppm ($C\underline{H}_2-CH_2-CH-S$). On retrouve les signaux à 5,25ppm ($C\underline{H}-OCO$), 4,26 et 4,16ppm ($C\underline{H}_2-OCO$), 2,29ppm ($C\underline{H}_2-CO$), 1,60ppm ($C\underline{H}_2-CH_2-CO$), 1,26ppm ($CH_2-C\underline{H}_2-CH_2$), 0,87ppm ($C\underline{H}_3-CH_2$) caractéristisques de la structucture des triglycérides.

Le spectre FTIR (Spectre Infra Rouge à Transformée de Fourrier) du polyol permet de confirmer la disparition des doubles liaisons de l'huile de colza. La disparition de la bande d'absorption à 3008 $cm^{-1}$ (élongation CH sp2), ainsi que la diminution de celle à 721 $cm^{-1}$ (cis CC déformation) confirment la consommation des doubles liaisons de l'huile de colza.

Cette dernière ne disparait pas complètement du fait de la présence de la bande d'absorption $CH_2$ rocking centré sur la même valeur de nombre d'onde. On note également l'apparition puis la disparition d'une bande à 964 $cm^{-1}$ (trans CC déformation). L'apparition de la large bande à 3400 $cm^{-1}$ confirme la mise en place de fonctions OH. La formation des liaisons CS et la disparition des liaisons SH ne sont pas visibles par la technique FT-IR, étant donné la trop faible intensité des bandes d'absorption attendues à 600-700 $cm^{-1}$ et à 2500-2600 $cm^{-1}$.

Le dosage alcool permet de déterminer l'indice d'hydroxyle dans le cas de la fonctionnalisation des corps gras par un thiol porteur d'un groupement alcool. La méthode utilisée permet d'accéder au nombre de fonctions acides par unité de masse du polyol (exprimé en mg KOH/g d'échantillon). Environ 0,5g d'échantillon est pesé exactement et mélangé avec 10 mL d'une solution acétylée, contenant un mélange pyridine/anhydride acétique (88/12 en volume), dans un ballon de 50 mL muni d'un réfrigérant. Après deux heures de réactions à 100°C, le milieu réactionnel est refroidi à température ambiante puis hydrolysé par 100 mL d'eau glacée. Sous très forte agitation, 20 mL de toluène sont ajoutés et la solution est titrée avec une solution aqueuse de KOH (0,5 mol/L), la phénolphtaléine jouant le rôle d'indicateur coloré. Trois blancs sont effectués et l'indice d'hydroxyle est donné par la formule suivante :

$$I_{OH} = \frac{(V_{blanc} - V_{échantillo\ n}) \times 56,1 \times 0,5}{m_{échantillo\ n}} + I_a$$

Le dosage des fonctions acide carboxylique est effectué dans le but de calculer les valeurs exactes de l'indice d'hydroxyle. En effet, l'indice d'hydroxyle est basé sur un dosage acido-basique et la présence de fonctions acide implique une sous-estimation de cet indice. Ce dosage est effectué à l'aide d'une solution de potasse éthanolique, elle même étalonnée par une solution d'acide chlorhydrique.

$I_a$= 2,5 mg KOH/g

$I_{OH}$ = 187 mg KOH/g

La conversion des doubles liaisons atteint 98,5% et la fonctionnalité est d'environ f= 3,94

Le procédé mis en oeuvre sous irradiation UV, permet d'obtenir des corps gras fonctionnalisé avec un taux de fonctionnalisation élevé ainsi qu'un taux de conversion des doubles liaisons de l'huile végétale élevé.

Exemple 2 : Synthèse de polyuréthane à partir du polyol obtenu dans l'exemple 1

Le polyol synthétisé dans l'exemple 1 est mélangé au methylene diphenyl 4,4'-diisocyanate (MDI) en présence de dibutyltin dilaurate (DBTDL). Les quantités introduites sont données dans le tableau 2 ci dessous. Le mélange est porté à 120°C pendant 2 heures.

Tableau 2

| | Huile modifiée | MDI | DBTDL |
|---|---|---|---|
| fonctionnalité | 3.94 | 2 | - |
| Masse (g) | 2 | 0.83 | 0.02 |
| Pourcentage molaire (%) | 33.46 | 65.91 | 0.63 |

La réaction est quantitative, le rendement est de 100%.

Les corps gras fonctionnalisés permettent la synthèse de polymère, notamment polyuréthane.

Exemple 3: Synthèse d'un corps gras fonctionnalisé de type polyol par voie thermique à partir de l'huile de colza (ne faisant pas partie de l'invention)

Dans un ballon bicol de 50 mL surmonté d'un réfrigérant à eau sont introduits l'huile de colza (2g, $2,3.10^{-3}$mol), le mercaptoéthanol (3,6g, $4,6.10^{-2}$mol), le dioxane (0,7g, $7,5.10^{-3}$mol) et le tert-amyl peroxypivalate (0,25g, $1.10^{-3}$mol). Le milieu réactionnel est porté à 50°C et maintenu à cette température pendant 24 heures. Après synthèse, le produit est purifié par distillation sous pression réduite ($10^{-2}$ bars) et à faible température (50°C). Le polyol obtenu est un liquide visqueux orangé.

L'absence de signal à 5,40ppm ($C\underline{H}=C\underline{H}$) et à 2,00ppm ($C\underline{H}_2$-CH=) sur le spectre RMN $^1H$ du polyol indique que les doubles liaisons de l'acide oléique ont réagi. Ces disparitions s'accompagnent de l'apparition des signaux caractéristiques de l'addition du mercaptoéthanol, avec notamment l'apparition du multiplet à 2,5ppm caractéristique du proton appartenant au carbone asymétrique en alpha du soufre (CH-S) d'un multiplet à 1,53ppm ($C\underline{H}_2$-CH-S) et à 1,40ppm ($C\underline{H}_2$-$CH_2$-CH-S). On retrouve les signaux à 5,25ppm ($C\underline{H}$-OCO), 4,26 et 4,16ppm ($C\underline{H}_2$-OCO), 2,29ppm ($C\underline{H}_2$-CO), 1,60ppm ($C\underline{H}_2$-$CH_2$-CO), 1,26ppm ($CH_2$-$C\underline{H}_2$-$CH_2$), 0,87ppm ($C\underline{H}_3$-$CH_2$) caractéristisques de la structucture des triglycérides.

La disparition de la bande d'absorption à 3008 $cm^{-1}$ (élongation CH sp2), ainsi que la diminution de celle à 721 $cm^{-1}$ (cis CC déformation) confirment la consommation des doubles liaisons. L'apparition de la large bande à 3400 $cm^{-1}$ confirme la mise en place de fonctions OH.

$I_a$= 2,5 mg KOH/g

$I_{OH}$ = 182 mg KOH/g

La conversion des doubles liaisons atteint 96% et la fonctionnalité est d'environ f= 3,84. Le procédé mis en oeuvre à 50°C, permet d'obtenir des corps gras fonctionnalisé avec un taux de fonctionnalisation élevé ainsi qu'un taux de conversion des doubles liaisons de l'huile végétale de départ élevé.

Exemple 4 : Synthèse d'un corps gras fonctionnalisé de type diol gras par voie photochimique à partir d'acide oléique (ne faisant pas partie de l'invention)

La réaction d'estérification est effectuée dans un ballon bicol de 100 mL surmonté d'un appareillage de type Dean Stark. Une fois l'éthylène glycol (4,4g, 0,071 mol), l'acide oléique (4g, 0,014mol), l'acétone (8g) et l'acide méthanesulfonique (0,16g, $1,6.10^{-3}$mol) introduits, le milieu réactionnel est porté à 85°C pendant 6 heures, du solvant étant ajouté régulièrement. Après synthèse, le solvant est éliminé par distillation puis le produit est purifié par extraction liquide-liquide avec un système acétate d'éthyl / eau saturée en chlorure de sodium. La phase organique est alors séchée par un agent desséchant de type sulfate de magnésium, puis concentrée à l'évaporateur rotatif sous vide à 40°C. Le produit est obtenu sous forme d'un liquide visqueux brun.

Dans un tube en quartz de 10 mL sont introduits l'ester oléique précédemment synthétisé (2,5g, $7,6.10^{-3}$mol) et le β-mercaptoéthanol (1,8g, $2,3.10^{-2}$mol). Le milieu réactionnel est placé sous irradiation UV d'une lampe mercure équipée d'un filtre 250-450 nm, délivrant une intensité de 15000mW/$cm^2$. Une forte agitation est maintenue pendant toute la durée de la réaction. Après 2 heures d'exposition, le milieu réactionnel est dilué dans l'acétate d'éthyle et l'excès de β-mercaptoéthanol est lavé trois fois avec une solution aqueuse de chlorure de sodium. La phase organique est alors séchée par un agent desséchant de type sulfate de magnésium, puis concentrée à l'évaporateur rotatif sous vide à 40°C. Le diol est obtenu sous forme d'un liquide visqueux brun.

Les analyses effectuées sont les mêmes que dans le cas de la synthèse d'un polyol.

Le spectre RMN $^1H$ du diol présente les signaux à 4,20ppm ($C\underline{H}_2$-OCO), 3,84ppm ($C\underline{H}_2$-OH) indiquant que l'estérification a bien eu lieu. L'absence de signal à 5,40ppm ($C\underline{H}=C\underline{H}$) et à 2,00ppm ($C\underline{H}_2$-CH=) indique que les doubles liaisons de l'acide oléique ont réagi. Ces disparitions s'accompagnent de l'apparition des signaux caractéristiques de l'addition du mercaptoéthanol, avec notamment l'apparition du multiplet à 2,5ppm caractéristique du proton appartenant au carbone asymétrique en alpha du soufre ($C\underline{H}$-S) d'un multiplet à 1,51ppm ($C\underline{H}_2$-CH-S) et à 1,38ppm ($C\underline{H}_2$-$CH_2$-CH-S). On retrouve les signaux à 2,29ppm ($C\underline{H}_2$-CO), 1,60ppm ($C\underline{H}_2$-$CH_2$-CO), 1,26ppm ($CH_2$-$C\underline{H}_2$-$CH_2$), 0,87ppm ($C\underline{H}_3$-$CH_2$) caractéristisques de la structucture des acides gras.

$I_a$= 29 mg KOH/g

$I_{OH}$ = 259 mg KOH/g

La conversion des fonctions acides en ester atteint 85%, la conversion des doubles liaisons atteint 99% et la fonctionnalité est d'environ f= 1,85.

Le procédé mis en oeuvre sous rayonnement UV, permet d'obtenir des corps gras fonctionnalisé avec un taux de fonctionnalisation élevé ainsi qu'un taux de conversion des doubles liaisons de l'acide gras de départ élevé.

Exemple 5 : Synthèse d'un corps gras fonctionnalisé de type diol par voie photochimique à partir d'huile de colza (ne faisant pas partie de l'invention)

Dans un ballon bicol de 50 mL surmonté d'un appareil Dean Stark sont introduits les esters méthyliques d'huile de colza (1,5g, $5{,}0.10^{-3}$mol) et l'éthanolamine (0,4g, $6{,}3.10^{-3}$mol). Le milieu réactionnel est porté à 100°C et maintenu sous agitation pendant 15 heures. Le milieu réactionnel est refroidi puis solubilisé dans l'acétate d'éthyle, lavé trois fois avec une solution aqueuse de chlorure de sodium. La phase organique est alors séchée par un agent desséchant de type sulfate de magnésium, puis concentrée à l'évaporateur rotatif sous vide à 40°C. Le produit est obtenu sous forme solide orangé.
Dans un tube en quartz de 10 mL sont introduits l'amide grasse précédemment synthétisée (1g, $3{,}1.10^{-3}$mol) et le mercaptoéthanol (2,6g, $3{,}4.10^{-2}$mol), le mélange est homogénéisé par léger chauffage. Le milieu réactionnel est placé sous irradiation UV d'une lampe mercure équipée d'un filtre 250-450 nm, délivrant une intensité de 15000mW/cm$^2$. Une forte agitation est maintenue pendant toute la durée de la réaction.
Après 5 heures d'exposition, le milieu réactionnel est dilué dans l'acétate d'éthyle et l'excès de mercaptoéthanol est lavé trois fois avec une solution aqueuse d'hydroxyde de sodium. La phase organique est alors séchée par un agent desséchant de type sulfate de magnésium, puis concentrée à l'évaporateur rotatif sous vide à 40°C. Le diol gras est obtenu sous forme d'un solide orangé.
Le signal à 3.43ppm, caractéristique des protons en alpha de l'amide ($C\underline{H}_2$-NHCO) indique que l'amidification a effectivement eu lieu. L'absence de signal à 5,40ppm ($C\underline{H}$=$C\underline{H}$) et à 2,00ppm ($C\underline{H}_2$-CH=) sur le spectre RMN $^1$H du produit final indique que les doubles liaisons ont réagi. Ces disparitions s'accompagnent de l'apparition des signaux caractéristiques de l'addition du mercaptoéthanol, avec notamment l'apparition du multiplet à 2,58ppm caractéristique du proton appartenant au carbone asymétrique en alpha du soufre (CH-S), ainsi que l'appartition d'un multiplet à 1,52ppm ($C\underline{H}_2$-CH-S) et à 1,40ppm ($C\underline{H}_2$-$CH_2$-CH-S). On retrouve les signaux à 2,21ppm ($C\underline{H}_2$-CO), 1,63ppm ($C\underline{H}_2$-$CH_2$-CO), 1,26ppm ($CH_2$-$C\underline{H}_2$-$CH_2$), 0,88ppm ($C\underline{H}_3$-$CH_2$) caractéristisques de la structure de l'amide grasse formée.
$I_{OH}$=268 mgKOH/g
La conversion des fonctions ester en amide atteint 100%, la conversion des doubles liaisons atteint 95% et la fonctionnalité est d'environ f= 1,95.

Le procédé mis en oeuvre sous rayonnement UV, permet d'obtenir des corps gras fonctionnalisé avec un taux de fonctionnalisation élevé ainsi qu'un taux de conversion des doubles liaisons de l'huile végétale ou de l'acide gras de départ élevé.

Exemple 6: Synthèse d'un corps gras fonctionnalisé de type diamine grasse par voie photochimique à partir de méthyl oléate

L'amidification est réalisée dans un ballon bicol de 100 mL surmonté d'un appareil Dean Stark. Le méthyle oléate (1,0g, $3{,}4.10^{-3}$mol), la 1,3-diaminopropane (0,6g, $8{,}1.10^{-3}$mol) sont maintenus à 100°C sous agitation pendant 48 heures. (Le milieu réactionnel est refroidi puis solubilisé dans l'acétate d'éthyle, lavé trois fois avec une solution aqueuse de chlorure de sodium. La phase organique est alors séchée par un agent desséchant de type sulfate de magnésium, puis concentrée à l'évaporateur rotatif sous vide à 40°C. Le produit est obtenu sous forme solide légèrement jaune.
Dans un tube en quartz de 10 mL sont introduits l'amide grasse précédemment synthétisée (1g, $3{,}1.10^{-3}$mol), l'éthanol (3,7g) et la cystéamine (1,2g, $1{,}1.10^{-2}$mol). Le milieu réactionnel est placé sous irradiation UV d'une lampe mercure équipée d'un filtre 250-450 nm, délivrant une intensité de 15000mW/cm$^2$. Une forte agitation est maintenue pendant toute la durée de la réaction. Après 5 heures d'exposition, le milieu réactionnel est dilué dans l'acétate d'éthyle et l'excès de cystéamine est lavé trois fois avec une solution aqueuse d'hydroxyde de sodium. La phase organique est alors séchée par un agent desséchant de type sulfate de magnésium, puis concentrée à l'évaporateur rotatif sous vide à 40°C. La diamine grasse est obtenue sous forme d'un solide orangé.
Outre les analyses RMN 1H et FTIR précédemment décrites, le dosage amine permet de vérifier la fonctionnalisation des corps gras par un thiol porteur d'un groupement amine.
La présence sur le spectre RMN $^1$H de la diamine des signaux à 3,30ppm ($C\underline{H}_2$-NHCO), 2,85ppm ($C\underline{H}_2$-$NH_2$) ainsi que la disparition du signal à 3,66ppm caractéristique de l'ester méthylique de départ ($C\underline{H}_3$-OCO) indiquent que l'amidification a bien eu lieu. L'absence de signal à 5,40ppm ($C\underline{H}$=$C\underline{H}$) et à 2,00ppm ($C\underline{H}_2$-CH=) indique que les doubles liaisons de l'ester methyllique de départ ont réagi. Ces disparitions s'accompagnent de l'apparition des signaux caractéristiques de l'addition de la cystéamine, avec notamment l'apparition du multiplet à 2,57ppm caractéristique du proton appartenant au carbone asymétrique en alpha du soufre ($C\underline{H}$-S), d'un multiplet à 1,53ppm ($C\underline{H}_2$-CH-S) et à 1,39ppm ($C\underline{H}_2$-$CH_2$-CH-S). On retrouve les signaux à 2,20ppm ($C\underline{H}_2$-CONH), 1,60ppm ($C\underline{H}_2$-$CH_2$-CO), 1,26ppm ($CH_2$-$C\underline{H}_2$-$CH_2$), 0,87ppm ($C\underline{H}_3$-$CH_2$) caractéristisques de la structure des acides gras.
Le dosage des fonctions amine est basé sur un dosage acido-basique. L'échantillon est solubilisé dans l'eau distillée

acidifiée par HCl, Le pH de la solution est abaissée par ajout d'acide chlorhydrique (1mol/L) jusqu'à une valeur de 1. La solution est ensuite dosée à l'aide d'une solution d'hydroxyde de sodium (0,5mol/L). Le dosage est suivi par pHmétrie, deux volumes équivalents sont détectés. L'indice d'amine est alors donné par la formule suivante :

$$I_{NH2}=\frac{\Delta V_{eq}\times 0,5}{m_{ech}}$$

$I_{NH2} = 4,2.10^{-3}$ mol/g
La conversion des fonctions ester en amide atteint 95%, la conversion des doubles liaisons atteint 80% et la fonctionnalité est d'environ f= 1,8.

Le procédé selon l'invention, mis en oeuvre sous rayonnement UV, permet d'obtenir des corps gras fonctionnalisé avec un taux de fonctionnalisation élevé ainsi qu'un taux de conversion des doubles liaisons de l'acide gras de départ élevé.

Exemple 7 : Synthèse de corps gras fonctionnalisé de type polyacide par voie thermique à partir d'huile de colza (ne faisant pas partie de l'invention)

Dans un ballon bicol de 100 mL surmonté d'un réfrigérant sont introduits l'huile de colza (2g, $2,3.10^{-3}$mol, soit environ $9,3.10^{-3}$mol d'insaturations), l'acide thioglycolique (1,1g, $1,1.10^{-2}$mol), et l'AIBN (0,2g, $1,3.10^{-3}$mol) solubilisé dans l'acétate d'éthyle (1,4g, $1,6.10^{-2}$mol). Le milieu réactionnel est porté à 40°C et maintenu sous forte agitation pendant 48 heures. Le milieu réactionnel est dilué dans l'acétate d'éthyle et l'excès d'acide est lavé trois fois avec une solution aqueuse de chlorure de sodium jusqu'à pH neutre des eaux de lavage. La phase organique est alors séchée par un agent desséchant de type sulfate de magnésium, puis concentré à l'évaporateur rotatif sous vide à 40°C. Le polyacide est obtenu sous forme d'un liquide visqueux orangé. L'absence de signal à 5,40ppm ($C\underline{H}=C\underline{H}$) et à 2,00ppm ($C\underline{H}_2-CH=$) sur le spectre RMN $^1H$ du produit final indique que les doubles liaisons de l'huile de colza ont réagi. Ces disparitions s'accompagnent de l'apparition des signaux caractéristiques de l'addition de l'acide thioglycolique, avec notamment l'apparition du multiplet à 2,76ppm caractéristique du proton appartenant au carbone asymétrique en alpha du soufre ($C\underline{H}-S$), ainsi que l'apparition du singulet à 3,22ppm ($C\underline{H}_2-S$), d'un multiplet à 1,53ppm ($C\underline{H}_2-CH-S$) et à 1,39ppm ($C\underline{H}_2-CH_2-CH-S$). On retrouve les signaux à 5,27ppm ($C\underline{H}-OCO$), 4,25 et 4,16ppm ($C\underline{H}_2-OCO$), 2,31ppm ($C\underline{H}_2-CO$), 1,59ppm ($C\underline{H}_2-CH_2-CO$), 1,26ppm ($CH_2-C\underline{H}_2-CH_2$), 0,87ppm ($C\underline{H}_3-CH_2$) caractéristiques de la structucture des triglycérides.
$I_a$= 145 mg KOH/g
La conversion des doubles liaisons atteint 80% et la fonctionnalité est d'environ f= 3,2.

Le procédé mis en oeuvre à 40°C, permet d'obtenir des corps gras fonctionnalisé avec un taux de fonctionnalisation élevé ainsi qu'un taux de conversion des doubles liaisons de l'huile végétale de départ élevé.

Exemple 8 : Synthèse de corps gras fonctionnalisé de type «diacides» par voie thermique à partir d'huile de colza (ne faisant pas partie de l'invention)

Dans un ballon bicol de 100 mL surmonté d'un réfrigérant sont introduits le produit de la saponification d'une huile de colza (1g, $3,5.10^{-3}$mol, soit environ $4,6.10^{-3}$mol d'insaturations) et l'acide thioglycolique (1,0g, $1,1.10^{-2}$mol), et l'AIBN (0,1g, $0,7.10^{-3}$mol) solubilisé dans l'acétate d'éthyle (0,7g, $7,9.10^{-3}$mol). Le milieu réactionnel est porté à 40°C et maintenu sous forte agitation pendant 48 heures. Le milieu réactionnel est dilué dans l'acétate d'éthyle et l'excès d'acide est lavé trois fois avec une solution aqueuse de chlorure de sodium jusqu'à pH neutre des eaux de lavage. La phase organique est alors séchée par un agent desséchant de type sulfate de magnésium, puis concentré à l'évaporateur rotatif sous vide à 40°C. Le diacide est obtenu sous forme d'un liquide visqueux orangé.

L'absence de signal à 5,40ppm ($C\underline{H}=C\underline{H}$) et à 2,00ppm ($C\underline{H}_2-CH=$) sur le spectre RMN $^1H$ du produit final indique que les doubles liaisons de l'huile de colza ont réagi. Ces disparitions s'accompagnent de l'apparition des signaux caractéristiques de l'addition de l'acide thioglycolique, avec notamment l'apparition du multiplet à 2,76ppm caractéristique du proton appartenant au carbone asymétrique en alpha du soufre ($C\underline{H}-S$), ainsi que l'appartition du singulet à 3,22ppm ($C\underline{H}_2-S$), d'un multiplet à 1,53ppm ($C\underline{H}_2-CH-S$) et à 1,39ppm ($C\underline{H}_2-CH_2-CH-S$). On retrouve les signaux à 2,31ppm ($C\underline{H}_2-CO$), 1,59ppm ($C\underline{H}_2-CH_2-CO$), 1,26ppm ($CH_2-C\underline{H}_2-CH_2$), 0,87ppm ($C\underline{H}_3-CH_2$) caractéristisques de la structucture des acides gras.
$I_a$= 192 mg KOH/g

La conversion des doubles liaisons atteint 86% et la fonctionnalité est d'environ f= 2,14. Le procédé mis en oeuvre à 40°C, permet d'obtenir des corps gras fonctionnalisé avec un taux de fonctionnalisation élevé ainsi qu'un taux de conversion des doubles liaisons de l'huile végétale de départ élevé.

Exemple 9 : Synthèse de résine époxy à partir des polyacides obtenus selon les exemples 7 et 8

L'acide gras fonctionnalisé et l'huile de colza fonctionnalisée aux exemples 7 et 8 sont mélangés au diglycidyl ether de bisphenol A (DGEBA) en présence d'imidazole. Les quantités introduites sont données dans le tableau 3. Le mélange est porté à 105°C pendant 24 heures. Le matériau obtenu est souple et d'aspect brillant.

Tableau 3

| | Huile modifiée | Acide gras modifié | DGEBA | Imidazole |
|---|---|---|---|---|
| Indice acide (mg KOH/g) | 144,9 | 192,3 | - | - |
| Masse (g) | 1,1 | 1,92 | 2,24 | 0,0262 |
| Pourcentage molaire (%) | 9,25 | 39,25 | 48,5 | 3 |

La réaction est quantitative, le rendement est de 100%.
Les corps gras fonctionnalisés de l'invention ou les corps gras susceptibles d'être obtenus par le procédé de l'invention permettent la synthèse de résine époxy.

Exemple 10 : Courbes ATG (5°C/min, 10°C/min et 20°C/min) d'un polyol commercial et d'un polyol obtenu par fonctionnalisation d'une huile végétale

La tenue thermique des polyols synthétisés par le procédé de l'invention et des polyols commerciaux a été analysée par ATG (Analyse ThermoGravimétrique), à l'aide d'un appareil TGA-Q50, TA Instruments, équipé d'une nacelle en platine, de coupelles en aluminium, sous flux constant d'azote avec une rampe de température entre 20°C et 500°C. Chaque polyol a été testé pour trois vitesses de chauffe : 5°C/min, 10°C/min, 20°C/min.
Les corps gras fonctionnalisés selon l'invention présentent une température de dégradation supérieure à celle des polyols commerciaux. Ainsi, les poyols de l'invention lorsqu'ils sont utilisés dans la synthèse d'autres polymères, permettent de limiter la quantité de COV dégagée.

## Revendications

**1.** Corps gras fonctionnalisé choisi parmi les composés de formules (IIa), (IIb) ou (IIc):

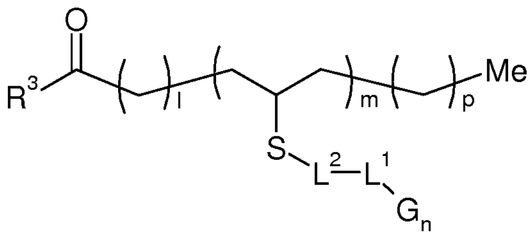

(IIa)

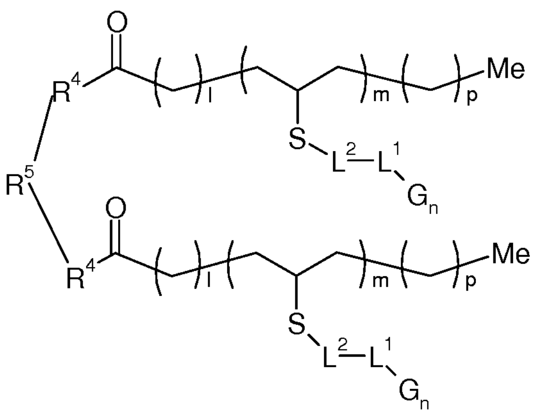

(IIb)

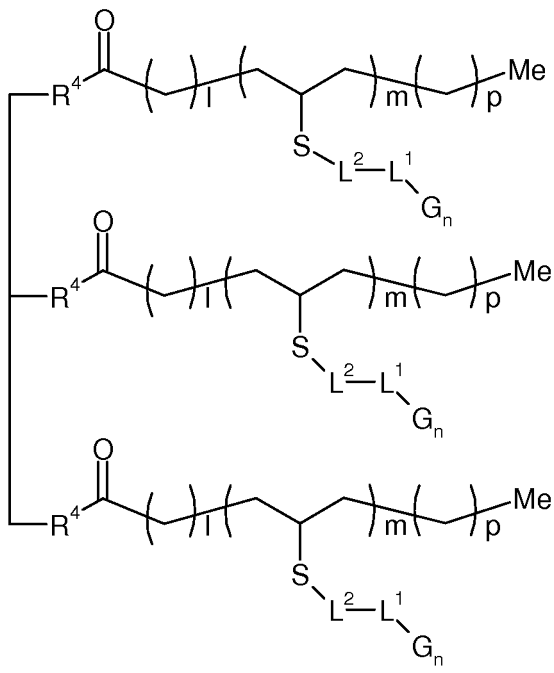

(IIc)

dans lesquelles :

- G, identique ou différent, représente $-NR^1H$ ; -C(O)OH ;
- n représente 1 ou 2 ;
- $R^1$ représente un atome d'hydrogène ; un radical alkyl, linéaire ou ramifié, en $C_1$-$C_{10}$, non substitué ou substitué par au moins un groupe choisi parmi -OH, -C(O)OH, $-NH_2$, - C(O)H,

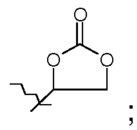 ;

- $L^1$ représente un groupe $-CH_2$ ; une liaison directe ;
- $L^2$ représente

  - un alkyle, linéaire ou ramifiée, en $C_1$-$C_{20}$, comprenant éventuellement un ou plusieurs hétéroatomes choisis parmi un atome d'oxygène, un atome d'azote, un atome de soufre, non-substitué ou substitué par au moins un groupe choisi parmi -OH, -C(O)OH, -C(O)H,

  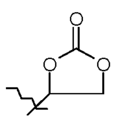 ,

  $-NHR^2$ avec $R^2$ représente un atome d'hydrogène ; un radical alkyle, linéaire ou ramifié, en $C_1$-$C_{10}$, non substitué ou substitué par au moins un groupe choisi parmi -OH, -C(O)OH, $-NH_2$, -C(O)H,

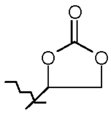 ;

- un carbo ou hétérocycle en $C_3$ à $C_8$, en particulier un groupe cycloaliphatique, un groupe aryle, un groupe hétéroaryle.

• $R^3$ représente :

- un groupe hydroxyle (OH),
- un groupe alkoxy de type -OX, avec X choisi parmi alkyl, linéaire ou ramifié, en $C_1$-$C_{20}$, éventuellement substitué par au moins un groupe choisi parmi -OH, -C(O)OH, - $NH_2$, -C(O)H,

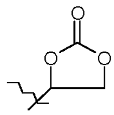 ;

- un groupe amine -NY'Y", avec Y' et Y", identiques ou différents représentent un atome d'hydrogène, un alkyl linéaire ou ramifié, en $C_1$-$C_{20}$, éventuellement substitué par au moins un groupe choisi parmi -OH, -C(O)OH, -$NH_2$, -C(O)H,

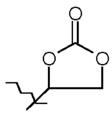 ;

- un groupe thio de type SZ, avec Z choisi parmi un alkyl linéaire ou ramifié, en $C_1$-$C_{20}$, éventuellement substitué par au moins un groupe choisi parmi -OH, -C(O)OH, -$NH_2$, - C(O)H,

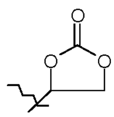 ;

• $R^4$ représente :

- un atome d'oxygène ;
- un atome d'azote ;

• $R^5$ représente :

- un groupe alkyl linéaire ou ramifié, en $C_1$-$C_{20}$, éventuellement substitué par au moins un groupe choisi parmi -OH, -C(O)OH, -$NH_2$, -C(O)H,

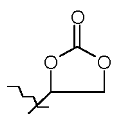 ;

• l représente un nombre entier compris entre 1 et 10 ;
• m représente un nombre entier compris entre 1 et 3 ;

• p représente un nombre entier compris entre 0 et 10

**2.** Procédé de préparation d'un corps gras fonctionnalisé selon la revendication 1 comprenant la réaction d'un corps gras d'origine naturelle choisi parmi

- les huiles végétales comprenant au moins deux insaturations et leurs dérivés,
- les acides gras comprenant au moins une insaturation et leurs dérivés,
- leurs mélanges

avec un dérivé thiol de formule (I)

$$G_nL^1\text{-}L^2\text{-}SH \qquad (I)$$

dans laquelle

• G, identique ou différent, représente $-NR^1H$ ; -C(O)OH ;
• n représente 1 ou 2 ;
• $R^1$ représente un atome d'hydrogène ; un radical alkyle, linéaire ou ramifié, en $C_1$-$C_{10}$, non substitué ou substitué par au moins un groupe choisi parmi -OH, -C(O)OH, $-NH_2$, - C(O)H,

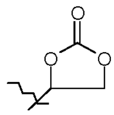 ;

• $L^1$ représente un groupe $-CH_2$ ; une liaison directe ;
• $L^2$ représente

- un alkyle, linéaire ou ramifiée, en $C_1$-$C_{20}$, comprenant éventuellement un ou plusieurs hétéroatomes choisis parmi un atome d'oxygène, un atome d'azote, un atome de soufre, non-substitué ou substitué par au moins un groupe choisi parmi -OH, - C(O)OH, -C(O)H,

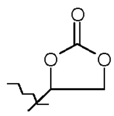 ,

$-NHR^2$ avec $R^2$ représente un atome d'hydrogène ; un radical alkyle, linéaire ou ramifié, en $C_1$-$C_{10}$, non substitué ou substitué par au moins un groupe choisi parmi -OH, -C(O)OH, $-NH_2$, -C(O)H,

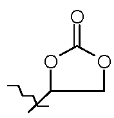 ;

- un carbo ou hétérocycle en $C_3$ à $C_8$, en particulier un groupe cycloaliphatique, un groupe aryle, un groupe hétéroaryle;

- sous action d'un rayonnement UV, ou
- sous action d'un rayonnement UV et en présence d'un photoamorceur.

**3.** Procédé selon la revendication 2 pour lequel l'huile végétale et ses dérivés comprennent entre 2 et 20 insaturations.

**4.** Procédé selon les revendications 2 ou 3 pour lequel l'huile végétale est choisie parmi les huiles végétales naturelles brutes ou purifiées et les huiles végétales issues de plantes ou cultures génétiquement modifiées.

**5.** Procédé selon la revendication 4 pour lequel l'huile végétale est choisie parmi l'huile de canola, l'huile de carthame, l'huile de colza, l'huile de coton, l'huile de lin, l'huile de maïs, l'huile de noisette, l'huile de noix de coco, l'huile d'olive, l'huile de palme, l'huile de pépins de raisins, l'huile de ricin, l'huile de sésame, l'huile de soja, l'huile de tournesol, seules ou en mélange.

**6.** Procédé selon la revendication 2 pour lequel les dérivés d'huiles végétales sont des esters gras obtenus par estérification ou transestérification des huiles végétales de la revendication 4, des amides grasses obtenues par amidification ou transamidification des huiles végétales de la revendication 4, des huiles partiellement époxydées.

**7.** Procédé selon la revendication 2 pour lequel les acides gras et leurs dérivés comprennent entre 1 et 6 insaturations.

**8.** Procédé selon les revendications 2 ou 7 pour lequel les acides gras et leurs dérivés sont choisis parmi l'acide arachidonique, l'acide docosahexaénoique, l'acide éicosapentaénoique, l'acide érucique, l'acide linoléique, l'acide linolénique, l'acide nervonique, l'acide oléique, l'acide palmitoléique, l'acide ricinoléique, l'acide vernolique; les esters d'acides gras ; les amides grasses obtenues par amidification des acides gras et les thioesters gras issus de la thioestérification des acides gras ; les acides gras obtenus à partir des huiles végétales choisies parmi l'huile de canola, l'huile de carthame, l'huile de colza, l'huile de coton, l'huile de lin, l'huile de maïs, l'huile de noisette, l'huile de noix de coco, l'huile d'olive, l'huile de palme, l'huile de pépins de raisins, l'huile de ricin, l'huile de sésame, l'huile de soja, l'huile de tournesol, seules ou en mélange; seuls ou en mélange.

**9.** Procédé selon les revendications 2 à 8 pour lequel G, identique ou différent, représente -C(O)OH, $-NH_2$.

**10.** Procédé selon les revendications 2 à 8 pour lequel le dérivé de thiol de formule (I) est choisi parmi la cystéamine (2-aminoethanethiol), l'acide thioglycolique (acide mercaptoacétique), l'acide mercaptosuccinique (acide thiomalic), l'acide 3-mercaptopropionique, la cystéine, 4-aminothiophenol, l'acide 6-mercaptohexanoique, l'acide 3-mercaptobenzoique, l'acide thiosalicylique,l'acide 4-mercaptophenylacetique, l'acide 8-mercaptooctanoique,l'acide 12-mercaptododecanoique.

**11.** Procédé selon les revendications 2 à 10 pour lequel la température est comprise entre la température ambiante et la température de dégradation totale du corps gras d'origine naturelle.

**12.** Procédé selon la revendication 11 pour lequel la température est comprise entre 40 °C et 250 °C.

**13.** Utilisation, pour la préparation d'un polymère, d'un corps gras fonctionnalisé selon la revendication 1.

**14.** Utilisation selon la revendication 13, pour la préparation d'un polymère choisi parmi les polyesters, polyamides, polycarbamates, polyisocyanates, les résines époxy.

**15.** Utilisation selon les revendications 13 ou 14, pour laquelle le polymère est préparé selon un procédé comprenant :

(a1) la préparation d'un corps gras fonctionnalisé selon les revendications 2 à 12 ;
(a2) la polycondensation entre le corps gras fonctionnalisé obtenu à l'étape (a1) et au moins une molécule au moins bifonctionnelle choisie parmi les isocyanates, alcools, acides carboxyliques, amines, carbamates, aldéhydes, époxy.

**16.** Utilisation selon les revendications 13 ou 14, pour laquelle le polymère est préparé selon un procédé comprenant:

(b1) la préparation d'un corps gras fonctionnalisé selon le procédé des revendications 2 à 12,
(b2) la préparation d'un second corps gras fonctionnalisé, selon le procédé des revendications 2 à 12, différent et au sein duquel la fonction G est différente de celle du corps gras préparé à l'étape (b1), puis
b3) la polycondensation entre les deux corps gras fonctionnalisés.

**Patentansprüche**

**1.** Funktionalisiertes Fett ausgewählt aus den Zusammensetzungen der Formeln (IIa), (IIb) oder (IIc):

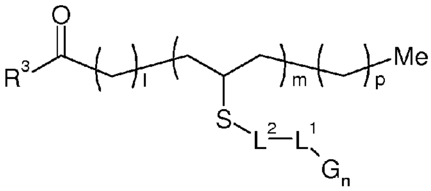

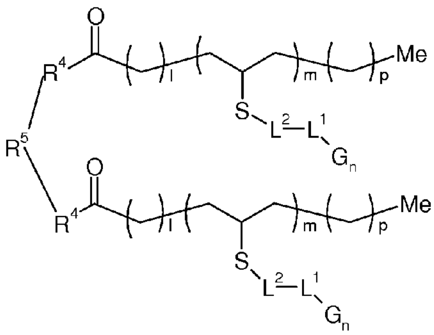

(IIa) (IIb)

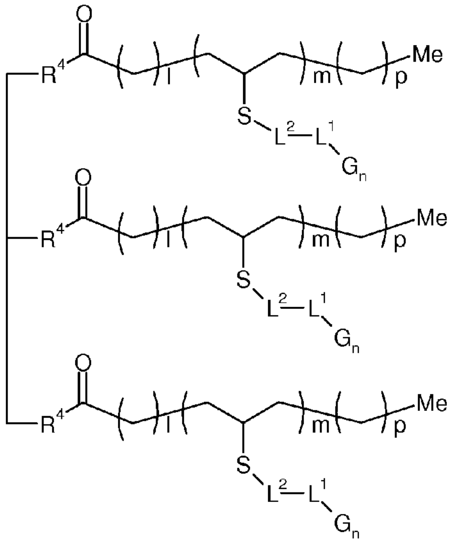

(IIc)

in denen:

- G, identisch oder unterschiedlich, $-NR^1H$; -C(O)OH darstellt;
- n 1 oder 2 darstellt;
- $R^1$ darstellt ein Wasserstoffatom, ein Alkylradikal, linear oder verzweigt, mit $C_1$-$C_{10}$, nicht substituiert oder substituiert durch mindestens eine Gruppe ausgewählt aus -OH, -C(O)OH, $-NH_2$, -C(O)H,

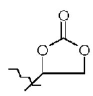 ;

- $L^1$ darstellt eine $-CH_2$ Gruppe; eine direkte Bindung;
- $L^2$ darstellt

  - ein Alkyl, linear oder verzweigt, mit $C_1$-$C_{20}$, das gegebenenfalls ein oder mehrere Heteroatome enthält, ausgewählt aus einem Sauerstoffatom, einem Stickstoffatom, einem Schwefelatom, nicht substituiert oder substituiert durch mindestens eine
  - Gruppe ausgewählt aus -OH, -C(O)OH, -C(O)H,

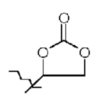 ,

-NHR² mit R² darstellend ein Wasserstoffatom; ein Alkylradikal, linear oder verstärkt, mit $C_1$-$C_{10}$,nicht substituiert oder substitiuiert durch mindestens eine Gruppe ausgewählt aus -OH, -Cd(O)OH, $-NH_2$, -C(O)H,

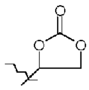
;

- ein Carbo- oder Heterozyklus mit $C_3$ bis $C_8$, insbesondere eine cykloalphatische Gruppe, eine Arylgruppe, eine Heteroarylgruppe,

• $R^3$ darstellt:

- eine Hydroxylgruppe (OH),
- eine Alkoxygruppe des Typs -OX, mit X ausgewählt aus Alkyl, linear oder verzweigt, mit $C_1$-$C_{20}$, gegebenenfalls substituiert durch mindestens eine Gruppe, ausgewählt aus -OH, -C(O)OH, - $NH_2$, -C(O)H,

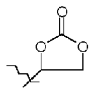
;

- eine Amingruppe - NY'Y", mit Y' und Y", identisch oder unterschiedlich, darstellend ein Wasserstoffatom, ein Alkyl, linear oder verzweigt, mit $C_1$-$C_{20}$, gegebenenfalls substituiert durch mindestens eine Gruppe ausgewählt aus -OH, -C(O)OH, $-NH_2$, -C(O)H,

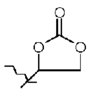
;

- eine Thiogruppe des Typs SZ, mit Z ausgewählt aus einem Alkyl, linear oder verzweigt, mit $C_1$-$C_{20}$, gegebenenfalls substituiert durch mindestens eine Gruppe ausgewählt aus -OH, -C(O)OH, $-NH_2$, -C(O)H,

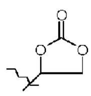
;

• $R^4$ darstellt:

- ein Sauerstoffatom;
- ein Stickstoffatom;

• $R^5$ darstellt:

- eine Alkylgruppe, linear oder verzweigt, mit $C_1$-$C_{20}$, gegebenenfalls substituiert durch mindestens eine Gruppe ausgewählt aus -OH, -C(O)OH, $-NH_2$, -C(O)H,

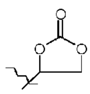
;

• l eine ganze Zahl zwischen 1 und 10 darstellt;
• m eine ganze Zahl zwischen 1 und 3 darstellt;
• p eine ganze Zahl zwischen 0 und 10 darstellt.

**2.** Verfahren zum Herstellen eines funktionalisierten Fettes nach Anspruch 1, die Reaktion eines Fettes natürlichen Ursprungs, ausgewählt aus

- pflanzlichen Ölen, die mindestens zwei ungesättigte Verbindungen und ihre Derivate enthalten,
- Fettsäuren, die mindestens eine ungesättigte Verbindung und ihre Derivate enthält,
- ihre Mischungen

mit einem Thiolderivat der Formel (I) umfassend

$$G_nL^1\text{-}L^2\text{-}SH \qquad (I)$$

in der

• G, identisch oder unterschiedlich,-$NR^1H$; -C(O)OH darstellt;
• n 1 oder 2 darstellt;
• $R^1$ darstellt ein Wasserstoffatom, ein Alkylradikal, linear oder verzweigt, mit $C_1$-$C_{10}$, nicht substituiert oder substituiert durch mindestens eine Gruppe ausgewählt aus -OH, -C(O)OH, -$NH_2$, -C(O)H,

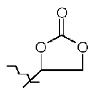;

• $L^1$ darstellt eine -$CH_2$ Gruppe; eine direkte Bindung;
• $L^2$ darstellt

- ein Alkyl, linear oder verzweigt, mit $C_1$-$C_{20}$, das gegebenenfalls ein oder mehrere Heteroatome enthält, ausgewählt aus einem Sauerstoffatom, einem Stickstoffatom, einem Schwefelatom, nicht substituiert oder substituiert durch mindestens eine
- Gruppe, ausgewählt aus -OH, -C(O)OH, -C(O)H,

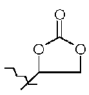,

-$NHR^2$ mit $R^2$ darstellend
ein Wasserstoffatom; ein Alkylradikal, linear oder verstärkt, mit $C_1$-$C_{10}$, nicht substituiert oder substituiert durch mindestens eine Gruppe, ausgewählt aus -OH, -Cd(O)OH, -$NH_2$, -C(O)H,

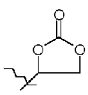;

- ein Carbo- oder Heterozyklus mit $C_3$ bis $C_8$, insbesondere eine cycloaliphatische Gruppe, eine Arylgruppe, eine Heteroarylgruppe,
- unter der Wirkung von UV-Strahlung, oder
- unter der Wirkung von UV-Strahlung in Anwesenheit eines Photoinitiators.

**3.** Verfahren nach Anspruch 2, für das das pflanzliche Öl und seine Derivate zwischen 2 und 20 ungesättigte Verbindungen enthalten.

**4.** Verfahren nach den Ansprüche 2 oder 3, für das das pflanzliche Öl ausgewählt ist aus natürlichen pflanzlichen Rohölen oder gereinigten Ölen und den pflanzlichen Ölen, die von Pflanzen oder genetisch modifizierten Kulturen stammen.

**5.** Verfahren nach Anspruch 4, für das das pflanzliche Öl ausgewählt ist aus Canolaöl, Saphloröl, Rapsöl, Baumwollsamenöl, Leinöl, Maisöl, Nussöl, Kokosnussöl, Olivenöl, Palmöl, Traubenkernöl, Rizinusöl, Sesamöl, Sojaöl, Sonnenblumenöl, allein oder in Mischung.

6. Verfahren nach Anspruch 2, für das die Derivate der pflanzlichen Öle Fettester, die durch Veresterung oder Umesterung der pflanzlichen Öle des Anspruchs 4 erhalten werden, Fettamide, die durch Amidierung oder Transamidierung der pflanzlichen Öle des Anspruchs 4 erhalten werden, teilweise epoxidierte Öle sind.

7. Verfahren nach Anspruch 2, für das die Fettsäuren und ihre Derivate zwischen 1 und 6 ungesättigte Verbindungen enthalten.

8. Verfahren nach den Ansprüchen 2 oder 7, für das die Fettsäuren und ihre Derivate ausgewählt sind aus Arachidonsäure, Docosahexaensäure, Eicosapentaensäure, Erucasäure, Linolsäure, Linolensäure, Nervonsäure, Oleinsäure, Palmitonsäure, Rizinolsäure, Vernolsäure; Fettsäureestern; Fettamiden, die durch Amidierung der Fettsäuren erhalten werden, und Fettthioestern, die von der Thioesterung der Fettsäuren stammen; Fettsäuren, die aus pflanzlichen Ölen erhalten werden, ausgewählt aus Canolaöl, Saphloröl, Rapsöl, Baumwollsamenöl, Leinöl, Maisöl, Nussöl, Kokosnussöl, Olivenöl, Palmöl, Traubenkernöl, Rizinusöl, Sesamöl, Sojaöl, Sonnenblumenöl, allein oder in Mischung.

9. Verfahren nach den Ansprüchen 2 bis 8, für das G, identisch oder unterschiedlich, -C(O)OH, $-NH_2$ darstellt.

10. Verfahren nach den Ansprüchen 2 bis 8, für das das Thiolderivat der Formel (I) ausgewählt ist aus Cysteamin (2-Aminoethanthiol), Thioglycolsäure (Mercaptoessigsäure), Mercaptosuccinilsäure (Thiomalsäure), 3-Mercaptopropion-Säure, Cystein, 4-Aminothiophenol, 6-Mercaptohexanolsäure, 3-Mercaptobenzolsäure, Thiosalicylsäure, 4-Mercaptophenylacetsäure, 8-Mercaptooctanolsäure, 12-Mercaptododecanolsäure.

11. Verfahren nach den Ansprüchen 2 bis 10, für das die Temperatur zwischen der Umgebungstemperatur und der Temperatur des Gesamtabbaus des Fetts natürlichen Ursprungs liegt.

12. Verfahren nach Anspruch 11, für das für das die Temperatur zwischen 40°C und 250°C liegt.

13. Verwendung eines funktionalisierten Fettes nach Anspruch 1 für die Herstellung eines Polymers.

14. Verwendung nach Anspruch 13, für die Herstellung eines Polymers ausgewählt aus Polyestern, Polyamiden, Polycarbamaten, Polyisocyanaten, Epoxidharzen.

15. Verwendung nach den Ansprüchen 13 oder 14, für die das Polymer nach einem Verfahren hergestellt wird, das umfasst:

(a1) die Herstellung eines funktionalisierten Fettes nach den Ansprüchen 2 bis 12;
(a2) die Polykondensation zwischen dem funktionalisierten Fett, das im Schritt (a1) erhalten wird, und mindestens einem mindestens bifunktionellen Molekül, ausgewählt aus Isocyanaten, Alkoholen, Carboxylsäuren, Aminen, Carbamaten, Aldehyden, Epoxid.

16. Verwendung nach den Ansprüchen 13 oder 14, für die das Polymer nach einem Verfahren hergestellt wird, das umfasst:

(b1) die Herstellung eines funktionalisierten Fettes nach dem Verfahren der Ansprüche 2 bis 12,
(b2) die Herstellung eines zweiten funktionalisierten Fettes nach dem Verfahren der Ansprüche 2 bis 12, das unterschiedlich ist und in dem die Funktion G unterschiedlich zu der des Fettes ist, das bei Schritt (b1) hergestellt wird, dann
(b3) die Polykondensation zwischen den zwei funktionalisierten Fetten.

## Claims

1. A functionalized fatty substance chosen from among the compounds of formulas (IIa), (IIb) or (IIc):

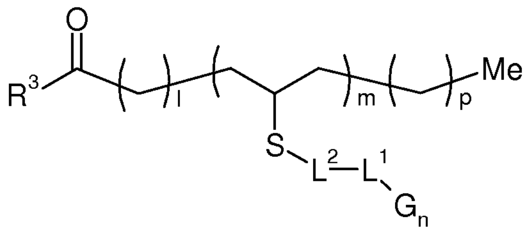

(IIa)

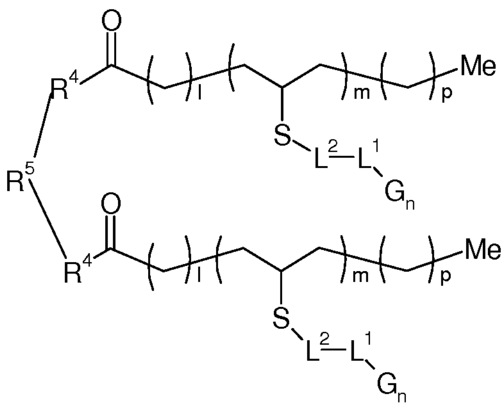

(IIb)

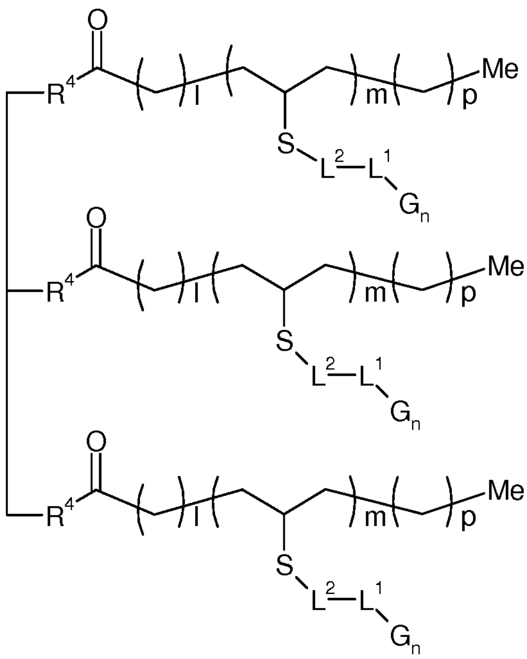

(IIc)

in which:

- G, the same or different, represents $-NR^1H$ ; -C(O)OH ;
- n is 1 or 2;
- $R^1$ is a hydrogen atom; a $C_1$-$C_{10}$ radical alkyl, linear or branched, non-substituted or substituted by at least one group chosen from among -OH, -C(O)OH, $-NH_2$,-C(O)H,

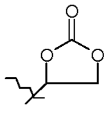 ;

- $L^1$ is a $-CH_2$ group ; a direct bond;
- $L^2$ is:

  - a $C_1$-$C_{20}$ alkyl, linear or branched, optionally comprising one or more heteroatoms chosen from among an oxygen atom, nitrogen atom, sulfur atom, non-substituted or substituted by at least one group chosen among -OH, -C(O)OH, -C(O)H,

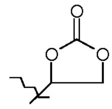
,

-NHR$^2$ where $R^2$ represents a hydrogen atom; a $C_1$-$C_{10}$ alkyl radical, linear or branched, non-substituted or substituted by at least one group chosen from among -OH, -C(O)OH, -$NH_2$, - C(O)H,

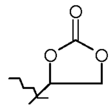
;

- a $C_3$ to $C_8$ carbo- or heterocycle, in particular a cycloaliphatic group, an aryl group, a heteroaryl group.

• $R^3$ is:

- a hydroxyl group (OH),
- an alkoxy group of -OX type, with X chosen from among a $C_1$-$C_{20}$ alkyl, linear or branched, optionally substituted by at least one group chosen from among -OH, -C(O)OH, - $NH_2$, -C(O)H,

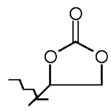
;

- a -NY'Y" amine group where Y' and Y", the same or different represent a hydrogen atom, a $C_1$-$C_{20}$ alkyl, linear or branched, optionally substituted by at least one group chosen from among -OH, -C(O)OH, -$NH_2$, -C(O)H,

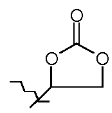
;

- a thio group of SZ type, with Z chosen from among a $C_1$-$C_{20}$ alkyl, linear or branched, optionally substituted by at least one group chosen from among -OH, -C(O)OH, -$NH_2$, -C(O)H,

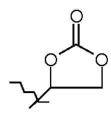
;

• $R^4$ is:

- an oxygen atom;
- a nitrogen atom;

• $R^5$ is:

- a $C_1$-$C_{20}$ alkyl group, linear or branched, optionally substituted by at least one group chosen from among -OH, -C(O)OH, -$NH_2$, -C(O)H,

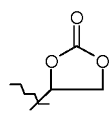
;

- l is an integer between 1 and 10;
- m is an integer between 1 and 3;
- p is an integer between 0 and 10.

**2.** A method for preparing a functionalized fatty substance according to claim 1 comprising the reaction of a fat of natural origin chosen from among:

- vegetable oils comprising at least two unsaturations and their derivatives;
- fatty acids comprising at least one unsaturation and their derivatives;
- the mixtures thereof

with a thiol derivative of formula (I)

$$G_nL^1\text{-}L^2\text{-}SH \qquad (I)$$

in which:

- G, the same or different, represents $-NR^1H$ ; $-C(O)OH$ ;
- n is 1 or 2;
- $R^1$ is a hydrogen atom; a $C_1$-$C_{10}$ alkyl radical non-substituted or substituted by at least one group chosen from among -OH, -C(O)OH, $-NH_2$, -C(O)H,

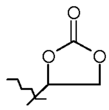
;

- $L^1$ is a $-CH_2$ group ; a direct bond;
- $L^2$ is :
  - a $C_1$-$C_{20}$ alkyl, linear or branched, optionally comprising one or more heteroatoms chosen from among an oxygen atom, a nitrogen atom, a sulfur atom, non-substituted or substituted by at least one group chosen from among -OH, -C(O)OH, - C(O)H,

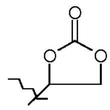
,

-$NHR^2$ where $R^2$ is a hydrogen atom; a $C_1$-$C_{10}$ alkyl radical, linear or branched, non-substituted or substituted by at least one group chosen from among -OH, - C(O)OH, $-NH_2$, -C(O)H,

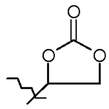
;

  - a $C_3$ to $C_8$ carbo- or heterocycle, in particular a cycloaliphatic group, an aryl group, a heteroaryl group;

- under the action of UV radiation, or
- under the action of UV radiation in the presence of a photoinitiator.

**3.** The method according to claim 2 in which the vegetable oil and the derivatives thereof comprise between 2 and 20 unsaturations.

**4.** The method according to claims 2 or 3 in which the vegetable oil is chosen from among natural crude or purified vegetable oils, and vegetable oils derived from genetically modified plants or cultures.

**5.** The method according to claim 4 in which the vegetable oil is chosen from among canola oil, safflower oil, rapeseed

oil, cottonseed oil, linseed oil, corn oil, hazelnut oil, coconut oil, olive oil, palm oil, grape-seed oil, castor oil, sesame oil, soybean oil, sunflower oil, alone or in a mixture.

**6.** The method according to claim 2 in which the derivatives of vegetable oils are fatty esters obtained by esterification or transesterification of the vegetable oils in claim 4, fatty amides obtained by amidification or transamidification of the vegetable oils in claim 4, partly epoxidated oils.

**7.** The method according to claim 2 in which the fatty acids and their derivatives comprise between 1 and 6 unsaturations.

**8.** The method according to claims 2 or 7 in which the fatty acids and their derivatives are chosen from among arachidonic acid, docosahexaenoic acid, eicosapentaenoic acid, erucic acid, linoleic acid, linolenic acid, nervonic acid, oleic acid, palmitoleic acid, ricinoleic acid, vernolic acid; the esters of fatty acids; the fatty amides obtained by amidification of fatty acids and the fatty thioesters derived from thioesterification of fatty acids; the fatty acids obtained from the vegetable oils chosen from among canola oil, safflower oil, rapeseed oil, cotton oil, linseed oil, corn oil, hazelnut oil, coconut oil, olive oil, palm oil, grape-seed oil, castor oil, sesame oil, soybean oil, sunflower oil, alone or in a mixture; alone or in a mixture.

**9.** The method according to claims 2 to 8 in which G, the same or different, represents -C(O)OH, $-NH_2$.

**10.** The method according to claims 2 to 8 in which the thiol derivative of formula (I) is chosen from among cysteamine (2-aminoethanethiol), thioglycolic acid (mercaptoacetic acid), mercaptosuccinic acid (thiomalic acid), 3-mercaptopropionic acid, cysteine, 4-aminothiophenol, 6-mercaptohexanoic acid, 3-mercaptobenzoic acid, thiosalicylic acid, 4-mercaptophenylacetic acid, 8-mercaptooctanoic acid, 12-mercaptododecanoic acid.

**11.** The method according to claims 2 to 10 in which the temperature is between ambient temperature and the total degradation temperature of the natural fatty substance.

**12.** The method according to claim 11 in which the temperature is between 40 °C and 250°C.

**13.** The use, for preparation of a polymer, of a functionalized fatty substance according to claim 1.

**14.** The use according to claim 13 for the preparation of a polymer chosen from among polyesters, polyamides, polycarbamates, polyisocyanates, epoxy resins.

**15.** The use according to claims 13 or 14 in which the polymer is prepared according to a method comprising:

(a1) preparation of a functionalized fatty substance according to claims 2 to 12;
(a2) polycondensation between the functionalized fatty substance obtained at step (a1) and at least one molecule, at least bifunctional, chosen from among isocyanates, alcohols, carboxylic acids, amines, carbamates, aldehydes, epoxy.

**16.** The use according to claims 13 or 14 in which the polymer is prepared according to a method comprising:

(b1) preparation of a functionalized fatty substance according to the method of claims 2 to 12;
(b2) preparation of a second functionalized fatty substance according to the method of claims 2 to 12, different and in which the function G is different from that of the fatty substance prepared at step (b1), then
(b3) polycondensation between the two functionalized fatty substances.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2006094227 A
- US 20060009365 A
- WO 02100991 A


### Littérature non-brevet citée dans la description


- **A.GUO et al.** *Journal of Polymers and The Environment,* 2002, vol. 10 (1/2), 49-52
- **SHARMA et al.** *Journal of Agricultural and Food Chemistry,* 2006, vol. 54 (26), 9866-9872
- **SAMUELSSON et al.** Part A : Polymer Chemistry. *Journal of Polymer Science,* 2004, vol. 42, 6346-6352
- **BANTCHEV et al.** *Journal of Agricultural and Food Chemistry,* 2009, vol. 57 (4), 1282-1290
- **LLUCH et al.** *Biomacromolecules,* 12 Mai 2010, vol. 11 (6
- **LOWE et al.** *polymer chemistry,* 25 Novembre 2009, vol. 1 (1
- **BANTCHEV et al.** *Journal of Adricultural and Food Chemistry,* 2009, vol. 57 (4